# EUROPÄISCHE PATENTSCHRIFT

(11) **EP 1 414 456 B1**
(45) Veröffentlichungstag und Bekanntmachung des Hinweises auf die Patenterteilung: **28.12.2011**
(21) Anmeldenummer: 02760242.4
(22) Anmeldetag: 12.07.2002
(51) Int. Cl.: A61K 31/4725, A61K 31/4412, A61K 31/4439, A61K 31/5377, C07D 401/12, C07D 417/12, C07D 413/12, C07D 211/76, A61P 7/02

(54) **PHENYLDERIVATE ALS FAKTOR XA INHIBITOREN**
PHENYL DERIVATIVES AS FACTOR XA INHIBITORS
DERIVES DE PHENYLE EN TANT QU'INHIBITEURS DU FACTEUR XA

(30) Priorität: 08.08.2001 DE 10139060
(43) Veröffentlichungstag der Anmeldung: 06.05.2004
(73) Patentinhaber: Merck Patent GmbH, 64293 Darmstadt (DE)
(72) Erfinder: DORSCH, Dieter, 64372 Ober-Ramstadt (DE); CEZANNE, Bertram, 64546 Mörfelden-Walldorf (DE); TSAKLAKIDIS, Christos, 69469 Weinheim (DE); MEDERSKI, Werner, 64673 Zwingenberg (DE); GLEITZ, Johannes, 64293 Darmstadt (DE); BARNES, Christopher, 65812 Bad Soden (DE)
(86) Internationale Anmeldenummer: PCT/EP2002/007798
(87) Internationale Veröffentlichungsnummer: WO 2003/013531

(56) Entgegenhaltungen:
- WO-A-00/71508
- WO-A-00/71510
- WO-A-00/71511
- WO-A-02/057236
- DE-A- 2 815 820
- US-A- 3 279 880
- DESAUBRY, LAURENT ET AL: "Synthesis of a conformationally constrained analog of BW A78U, an anticonvulsant adenine derivative" TETRAHEDRON LETTERS (1995), 36(24), 4249-52, XP004027961

## Beschreibung

Die Erfindung betrifft einzelne Verbindungen gemäß Anspruch 1 umfasst von der Formel I 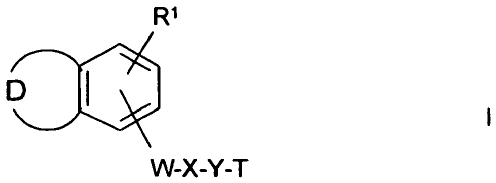 worin
- D: eine gesättigte, ganz oder teilweise ungesättigte 3- bis 4-gliedrige Alkylenkette bedeutet, bei der 1 bis 3 C-Atome durch N und/oder 1 bis 2 C-Atome durch 1 bis 2 O- und/oder 1 bis 2 S-Atome ersetzt sein können, wobei jedoch höchstens bis zu 3 C-Atome ersetzt werden und wobei zusätzlich eine ein-, zwei- oder dreifache Substitution der Alkylenkette und/oder eines darin befindlichen Stickstoffes durch Hal, A, -[C(R³)₂]ₙ-Ar, -[C(R³)₂]ₙ-Het, -[C(R³)₂]ₙ-Cycloalkyl, OR², N(R²)₂, NO₂, CN, COOR², CON(R²)₂, NR²COA, NR²SO₂A, COR², SO₂NR² und/oder S(O)ₘA auftreten kann, und wobei ferner auch eine CH₂-Gruppe der Alkylenkette durch eine C=O-Gruppe ersetzt sein kann,
- R¹: H, Hal, A, OR², N(R²)₂, NO₂, CN, COOR², CON(R²)₂, -[C(R³)₂]ₙ-Ar, -[C(R³)₂]ₙ-Het oder -[C(R³)]ₙ-Cycloalkyl,
- R²: H, A, -[C(R ³)₂]ₙ-Ar, -[C(R³)₂]ₙ-Het oder -[C(R³)₂]ₙ-Cycloalkyl,
- R³: H oder A,
- W: -C(R²)₂-, -[C(R²)₂]₂-, -OC(R²)₂- oder -NR²C(R²)₂-,
- X: CONR², CONR²C(R³)₂, -C(R³)₂NR² oder -C(R³)₂NR²C(R³)₂,
- Y: Alkylen, Cycloalkylen, Het-diyl oder Ar-diyl,
- T: einen ein- oder zweikernigen gesättigten oder ungesättigten Heterocyclus mit 1 bis 4 N-, O- und/oder S-Atomen, der ein-, zwei- oder dreifach durch Carbonylsauerstoff substituiert ist und ferner ein-, zwei- oder dreifach durch Hal, A, [C(R³)₂]ₙ-Ar, -[C(R³)₂]ₙ-Het, -[C(R³)₂]ₙ-Cycloalkyl, OR², N(R²)₂, NO₂, CN, COOR², CON(R²)₂, NR²COA, NR²CON(R²)₂, NR²SO₂A, COR², SO₂NR² und/oder S(O)ₘA substituiert sein kann,
- A: unverzweigtes oder verzweigtes Alkyl mit 1-6 C-Atomen, worin eine oder zwei CH₂-Gruppen durch O- oder S-Atome und/oder durch -CH=CH-Gruppen und/oder auch 1-7 H-Atome durch F ersetzt sein können,
- Ar: unsubstituiertes oder ein-, zwei- oder dreifach durch Hal, A, OR³, N(R³)₂, NO₂, CN, COOR³, CON(R³)₂, NR³COA, NR³CON(R³)₂, NR³SO₂A, COR³, SO₂N(R³)₂, S(O)ₘA substituiertes Phenyl, Naphthyl oder Biphenyl,
- Het: einen ein- oder zweikernigen gesättigten, ungesättigten oder aromatischen Heterocyclus mit 1 bis 4 N-, O- und/oder S-Atomen, der unsubstituiert oder ein-, zwei- oder dreifach durch Carbonylsauerstoff, Hal, A, -[C(R³)₂]ₙ-Ar, -[C(R³)₂]ₙ-Het¹, -[C(R³)₂]ₙ-Cycloalkyl, OR², N(R²)₂, NO₂, CN, COOR², CON(R²)₂, NR²COA, NR²CON(R²)₂, NR²SO₂A COR², SO₂NR² und/oder S(O)ₘA substituiert sein kann,
- Het¹: einen ein- oder zweikernigen gesättigten, ungesättigten oder aromatischen Heterocyclus mit 1 bis 2 N-, O- und/oder S-Atomen, der unsubstituiert oder ein- oder zweifach durch Carbonylsauerstoff, Hal, A, OR², N(R²)₂, NO₂, CN, COOR², CON(R²)₂, NR²COA, NR²CON(R²)₂, NR²SO₂A, COR², SO₂NR² und/oder S(O)ₘA substituiert sein kann,
- Hal: F, Cl, Br oder I,
- n: 0, 1⁻ oder 2,
- m: 0, 1 oder 2
bedeuten,
sowie ihre pharmazeutisch verwendbaren Salze, Solvate und Stereoisomere, einschließlich deren Mischungen in allen Verhältnissen.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die beanspruchten Verbindungen der Formel I gemäß Anspruch 1 und ihre Salze bei guter Verträglichkeit sehr wertvolle pharmakologische Eigenschaften besitzen. Insbesondere zeigen sie Faktor Xa inhibierende Eigenschaften und können daher zur Bekämpfung und Verhütung von thromboembolischen Erkrankungen wie Thrombose, myocardialem Infarkt, Arteriosklerose, Entzündungen, Apoplexie, Angina pectoris, Restenose nach Angioplastie und Claudicatio intermittens eingesetzt werden.

Die erfindungsgemäßen Verbindungen der Formel I gemäß Anspruch 1 können weiterhin Inhibitoren der Gerinnungsfaktoren Faktor VIIa, Faktor IXa und Thrombin der Blutgerinnungskaskade sein.

Aromatische Amidinderivate mit antithrombotischer Wirkung sind z.B. aus der EP 0 540 051 B1, WO 98/28269, WO 00/71508, WO 00/71511, WO 00/71493, WO 00/71507, WO 00/71509, WO 00/71512, WO 00/71515 oder WO 00/71516 bekannt. Cyclische Guanidine zur Behandlung thromboembolischer Erkrankungen sind z.B. in der WO 97/08165 beschrieben. Aromatische Heterocyclen mit Faktor Xa inhibitorischer Aktivität sind z.B. aus der WO 96/10022 bekannt. Substituierte N-[(Aminoiminomethyl)-phenylalkyl]-azaheterocyclylamide als Faktor Xa Inhibitoren sind in WO 96/40679 beschrieben.

Andere heterocyclische Faktor Xa - Inhibitoren sind auch in der WO 00/71510 A2 beschrieben.

Der antithrombotische und antikoagulierende Effekt der erfindungsgemäßen Verbindungen wird auf die inhibierende Wirkung gegenüber der aktivierten Gerinnungsprotease, bekannt unter dem Namen Faktor Xa, oder auf die Hemmung anderer aktivierter Serinproteasen wie Faktor VIIa, Faktor IXa oder Thrombin zurückgeführt.

Faktor Xa ist eine der Proteasen, die in den komplexen Vorgang der Blutgerinnung involviert ist. Faktor Xa katalysiert die Umwandlung von Prothrombin in Thrombin. Thrombin spaltet Fibrinogen in Fibrinmonomere, die nach Quervernetzung elementar zur Thrombusbildung beitragen. Eine Aktivierung von Thrombin kann zum Auftreten von thromboembolischen Erkrankungen führen. Eine Hemmung von Thrombin kann jedoch die in die Thrombusbildung involvierte Fibrinbildung inhibieren.

Die Messung der Inhibierung von Thrombin kann z.B. nach der Methode von G. F. Cousins et al. in Circulation 1996, 94, 1705-1712 erfolgen.

Eine Inhibierung des Faktors Xa kann somit verhindern, daß Thrombin gebildet wird.

Die erfindungsgemäßen Verbindungen der Formel I sowie ihre Salze greifen durch Inhibierung des Faktors Xa in den Blutgerinnungsprozeß ein und hemmen so die Entstehung von Thromben.

Die Inhibierung des Faktors Xa durch die erfindungsgemäßen Verbindungen und die Messung der antikoagulierenden und antithrombotischen Aktivität kann nach üblichen in vitro- oder in vivo-Methoden ermittelt werden. Ein geeignetes Verfahren wird z.B. von J. Hauptmann et al. in Thrombosis and Haemostasis 1990, 63, 220-223 beschrieben.

Die Messung der Inhibierung von Faktor Xa kann z.B. nach der Methode von T. Hara et al. in Thromb. Haemostas. 1994, 71, 314-319 erfolgen.

Der Gerinnungsfaktor VIIa initiiert nach Bindung an Tissue Faktor den extrinsischen Teil der Gerinnungskaskade und trägt zur Aktivierung des Faktors X zu Faktor Xa bei. Eine Inhibierung von Faktor Vlla verhindert somit die Entstehung des Faktors Xa und damit eine nachfolgende Thrombinbildung.

Die Inhibierung des Faktors VIIa durch die erfindungsgemäßen Verbindungen und die Messung der antikoagulierenden und antithrombotischen Aktivität kann nach üblichen in vitro- oder in vivo-Methoden ermittelt werden. Ein übliches Verfahren zur Messung der Inhibierung von Faktor VIIa wird z.B. von H. F. Ronning et al. in Thrombosis Research 1996, 84, 73-81 beschrieben.

Der Gerinnungsfaktor IXa wird in der intrinsischen Gerinnungskaskade generiert und ist ebenfalls an der Aktivierung von Faktor X zu Faktor Xa beteiligt. Eine Inhibierung von Faktor IXa kann daher auf andere Weise verhindern, daß Faktor Xa gebildet wird.

Die Inhibierung von Faktor IXa durch die erfindungsgemäßen Verbindungen und die Messung der antikoagulierenden und antithrombotischen Aktivität kann nach üblichen in vitro- oder in vivo-Methoden ermittelt werden. Ein geeignetes Verfahren wird z.B. von J. Chang et al. in Journal of Biological Chemistry 1998, 273, 12089-12094 beschrieben.

Die erfindungsgemäßen Verbindungen können weiterhin zur Behandlung von Tumoren, Tumorerkrankungen und/oder Tumormetastasen verwendet werden.

Ein Zusammenhang zwischen dem Tissuefaktor TF / Faktor VIIa und der Entwicklung verschiedener Krebsarten wurde von T.Taniguchi und N.R.Lemoine in Biomed. Health Res. (2000), 41 (Molecular Pathogenesis of Pancreatic Cancer), 57-59, aufgezeigt.

Die im nachfolgenden aufgeführten Publikationen beschreiben eine antitumorale Wirkung von TF-VII und Faktor Xa Inhibitoren bei verschiedenen Tumorarten:
K.M. Donnelly et al. in Thromb. Haemost. 1998; 79: 1041-1047;
E.G. Fischer et al. in J. Clin. Invest. 104: 1213-1221 (1999);
B.M. Mueller et al. in J. Clin. Invest. 101: 1372-1378 (1998);
M.E. Bromberg et al. in Thromb. Haemost. 1999; 82: 88-92

Die Verbindungen der Formel I gemäß Anspruch 1 können als Arzneimittelwirkstoffe in der Human- und Veterinärmedizin eingesetzt werden, insbesondere zur Behandlung und Verhütung von thromboembolischen Erkrankungen wie Thrombose, myocardialem Infarkt, Arteriosklerose, Entzündungen, Apoplexie, Angina pectoris, Restenose nach Angioplastie, Claudicatio intermittens, venöse Thrombose, pulmonale Embolie, arterielle Thrombose, myocardiale Ischämie, instabile Angina und auf Thrombose basierender Schlaganfall.

Die erfindungsgemäßen Verbindungen werden auch zur Behandlung oder Prophylaxe von atherosklerotischen Erkrankungen wie koronarer arterieller Erkrankung, cerebraler arterieller Erkrankung oder peripherer arterieller Erkrankung eingesetzt.

Die Verbindungen werden auch in Kombination mit anderen Thrombolytika bei myocardialem Infarkt eingesetzt, ferner zur Prophylaxe zur Reocclusion nach Thrombolyse, percutaner transluminaler Angioplastie (PTCA) und koronaren Bypass-Operationen.

Die erfindungsgemäßen Verbindungen werden ferner verwendet zur Prävention von Rethrombose in der Mikrochirurgie, ferner als Antikoagulantien im Zusammenhang mit künstlichen Organen oder in der Hämodialyse.

Die Verbindungen finden ferner Verwendung bei der Reinigung von Kathetern und medizinischen Hilfsmitteln bei Patienten *in vivo*, oder als Antikoagulantien zur Konservierung von Blut, Plasma und anderen Blutprodukten *in vitro.* Die erfindungsgemäßen Verbindungen finden weiterhin Verwendung bei solchen Erkrankungen, bei denen die Blutkoagulation entscheidend zum Erkrankungsverlauf beiträgt oder eine Quelle der sekundären Pathologie darstellt, wie z.B. bei Krebs einschließlich Metastasis, entzündlichen Erkrankungen einschließlich Arthritis, sowie Diabetes.

Die erfindungsgemäßen Verbindungen finden weiterhin Verwendung zur Behandlung von Migräne (F.Morales-Asin et al., Headache, 40, 2000, 45-47).

Bei der Behandlung der beschriebenen Erkrankungen werden die erfindungsgemäßen Verbindungen auch in Kombination mit anderen thrombolytisch wirksamen Verbindungen eingesetzt, wie z.B. mit dem "tissue plasminogen activator" t-PA, modifiziertem t-PA, Streptokinase oder Urokinase. Die erfindungsgemäßen Verbindungen werden mit den anderen genannten Substanzen entweder gleichzeitig oder vorher oder nachher gegeben.

Besonders bevorzugt ist die gleichzeitige Gabe mit Aspirin, um ein Neuauftreten der Thrombenbildung zu verhindern.

Die erfindungsgemäßen Verbindungen werden auch verwendet in Kombination mit Blutplättchen-Glycoprotein-Rezeptor (IIb/IIIa)-Antagonisten, die die Blutplättchenaggregation inhibieren.

Die Herstellung der Verbindungen der Formel I gemäß Anspruch 1 und ihrer Salze ist dadurch gekennzeichnet, daß man
a) zur Herstellung einer Verbindung der Formel I,
   worin W -OC(R²)₂- oder -NR²C(R²)₂- bedeutet,
   eine Verbindung der Formel II 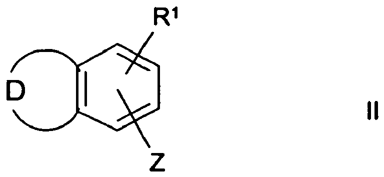 worin
   - Z: OH oder NHR² bedeutet
   und R¹, R² und D die in Anspruch 1 entsprechenden Bedeutungen haben, mit der Maßgabe, daß falls eine weitere OH- und/oder Aminogruppe vorliegt, diese geschützt ist,
   mit einer Verbindung der Formel III

   L-C(R²)₂-X-Y-T III

   worin
   L Cl, Br oder I bedeutet und R², X, Y und T die in Anspruch 1 entsprechenden Bedeutungen haben,
   umsetzt,
   und anschließend gegebenenfalls eine Schutzgruppe abspaltet,
b) zur Herstellung einer Verbindung der Formel I,
   - worin X: CONR² oder CONR²C(R³)₂ bedeutet,
   eine Verbindung der Formel IV 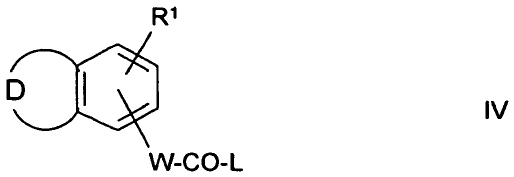 worin
   - L: Cl, Br, I oder eine freie oder reaktionsfähig funktionell
   abgewandelte OH-Gruppe bedeutet bedeutet
   und R¹, W und D die in Anspruch 1 entsprechenden Bedeutungen haben,
   mit der Maßgabe, daß falls eine weitere OH- und/oder Aminogruppe vorliegt, diese geschützt ist,
   mit einer Verbindung der Formel V

   Z'-Y-T V

   worin
   - Z': NHR² oder NHR²C(R³)₂ bedeutet
   und R², Y und T die in Anspruch 1 entsprechenden Bedeutungen haben, umsetzt,
   und anschließend gegebenenfalls eine Schutzgruppe abspaltet,
und/oder
eine Base oder Säure der Formel I gemäß Anspruch 1 in eines ihrer Salze umwandelt.

Gegenstand der Erfindung sind auch die optisch aktiven Formen (Stereoisomeren), die Enantiomeren, die Racemate, die Diastereomeren sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Unter pharmazeutisch verwendbaren Derivaten versteht man z.B. die Salze der erfindungsgemäßen Verbindungen als auch sogenannte Prodrug-Verbindungen.

Unter Prodrug-Derivaten versteht man mit z. B. Alkyl- oder Acylgruppen, Zuckern oder Oligopeptiden abgewandelte Verbindungen der Formel I, die im Organismus rasch zu den wirksamen erfindungsgemäßen Verbindungen gespalten werden.

Hierzu gehören auch bioabbaubare Polymerderivate der erfindungsgemäßen Verbindungen, wie dies z. B. in Int. J. Pharm. 115, 61-67 (1995) beschrieben ist.

Gegenstand der Erfindung sind auch Mischungen der erfindungsgemäßen Verbindungen, z.B. Gemische zweier Diastereomerer z.B. im Verhältnis 1:1, 1:2, 1:3, 1:4, 1:5, 1:10, 1:100 oder 1:1000.

Besonders bevorzugt handelt es sich dabei um Mischungen stereoisomerer Verbindungen.

Für alle Reste, die mehrfach auftreten, wie z.B. A, gilt, daß deren Bedeutungen unabhängig voneinander sind.

Vor- und nachstehend haben die Reste bzw. Parameter Y, T, W, R¹, R² die bei der Formel I entsprechenden Bedeutungen, falls nicht ausdrücklich etwas anderes angegeben ist.

D bedeutet vorzugsweise -CO-NH-CO, -CO-NH-CH₂-, -NH-CH=CH-, -O-CH=CH-, -N=CH-O-, -N=CH-NH-, -NH-NH-CO-, -NH-N=N-, -NH-CO-CH₂-, -NH-CO-O-, -N=CH-S-, -NH-CO-S-, -NH-CO-NH-, -O-NH-CO-, -NH-O-CO-, -N=CH-CH=CH-, -CH=N-CH=CH-, -N=N-CH=CH-, -N=CH-N=CH-, -N=CH-CH=N-, -N=N-N=CH-, -NH-CO-CH=CH-, -NH-CH=CH-CO-, -NH-CO-CH₂-CH₂-, -NH-CH₂-CH₂-CO-, -NH-CO-N=CH-, -N=CH-NH-CO-, -NH-CO-NH-CO-, -NH-CO-NH-CH₂-, -CH=N-N=CH-, -N⁻-S⁺=-N-, -O-CH₂-O-, ferner -CH=N-NH-CO-, -CH=CH-NH-, -NH-N=CH-, -O-CH₂CH₂-O-, -CO-NH-NH-CO-, -N=N-NH-CO-, -O-CO-NH-CH₂- oder -O-CO-NH-CO-, weiterhin **-**(CH₂)₃- oder -(CH₂)₄-.

D bedeutet besonders bevorzugt -CH=N-CH=CH-, -O-CH₂-O-, -CH=CH-NH-, -S-CH=N-, -NH-N=CH-, -O-CH₂CH₂-O-, -N=CH-CH=N- oder -N⁻-S⁺=-N-.

D kann ein- zwei- oder dreifach substituiert sein, vorzugsweise durch Hal, A, OR² oder N(R²)₂, auftreten kann, und/oder es kann auch eine CH₂-Gruppe der Alkylenkette durch eine C=O-Gruppe ersetzt sein. Ganz besonders bevorzugt ist eine einfach-Substitution durch A oder NH₂.

A bedeutet Alkyl, ist unverzweigt (linear) oder verzweigt, und hat 1, 2, 3, 4, 5, 6, 7, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2- , 3- oder 4-Methylpentyl, 1,1- , 1,2- , 1,3- , 2,2- , 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiter bevorzugt z.B. Trifluormethyl.

A bedeutet ganz besonders bevorzugt Alkyl mit 1-6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl.

Cycloalkyl bedeutet vorzugsweise Cyclopropyl, Cyclobutyl, Cylopentyl, Cyclohexyl oder Cycloheptyl.

Alkylen bedeutet vorzugsweise Methylen, Ethylen, Propylen, Butylen, Pentylen oder Hexylen, ferner verzweigtes Alkylen. -COA (Acyl) bedeutet vorzugsweise Acetyl, Propionyl, ferner auch Butyryl, Pentanoyl, Hexanoyl oder z.B. Benzoyl.

Hal bedeutet vorzugsweise F, Cl oder Br, aber auch I.

R¹ bedeutet vorzugsweise H.

W bedeutet vorzugsweise -C(R^{2'})₂-, -[C(R^{2'})₂]₂-, -OC(R^{2'})₂- oder -NR^{2'}C(R^{2'})₂-,
- worin R^{2'}: H, A' oder Phenyl,
- und A': Alkyl mit 1, 2, 3, 4, 5 oder 6 C-Atomen bedeutet.

X bedeutet vorzugsweise CONH, CONHCH₂, CH₂NH oder CH₂NHCH₂, ganz besonders bevorzugt CONH.

Y bedeutet vorzugsweise Alkylen oder Ar-diyl, besonders bevorzugt Methylen, Ethylen, Propylen oder unsubstituiertes oder einfach durch A oder F substituiertes 1,4-Phenylen, ferner auch Pyridin-diyl, vorzugsweise Pyridin-2,5-diyl. Y bedeutet insbesondere unsubstituiertes oder einfach durch Methyl, Ethyl oder Propyl substituiertes 1,3- oder 1,4-Phenylen.

Ar bedeutet z.B. unsubstituiertes Phenyl, Naphthyl oder Biphenyl, weiterhin vorzugsweise z.B. durch A, Fluor, Chlor, Brom, Iod, Hydroxy, Methoxy, Ethoxy, Propoxy, Butoxy, Pentybxy, Hexyloxy, Nitro, Cyan, Formyl, Acetyl, Propionyl, Trifluormethyl, Amino, Methylamino, Ethylamino, Dimethylamino, Diethylamino, Benzyloxy, Sulfonamido, Methylsulfonamido, Ethylsulfonamido, Propylsulfonamido, Butylsulfonamido, Dimethylsulfonamido, Phenylsulfonamido, Carboxy, Methoxycarbonyl, Ethoxycarbonyl, Aminocarbonyl mono-, di- oder trisubstituiertes Phenyl, Naphthyl oder Biphenyl.

Het bedeutet z.B. 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder-5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder - 5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7- Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, weiter bevorzugt 1,3-Benzodioxol-5-yl, 1,4-Benzodioxan-6-yl, 2,1,3-Benzothiadiazol-4- oder -5-yl oder 2,1,3-Benzoxadiazol-5-yl.

Die heterocyclischen Reste können auch teilweise oder vollständig hydriert sein.

Het kann also z. B. auch bedeuten 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3- oder -4-pyridyl, 1,2,3,4-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder - 4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-,-2-,-3-, -4-, -5-, -6-, -7- oder -8-isochinolyl, 2-, 3-, 5-, 6-, 7- oder 8- 3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydro-benzofuran-5- oder 6-yl, 2,3-(2-Oxo-methylendioxy)-phenyl oder auch 3,4-Dihydro-2H-1,5-benzodioxepin-6- oder-7-yl, ferner bevorzugt 2,3-Dihydrobenzofuranyl oder 2,3-Dihydro-2-oxo-furanyl.

T bedeutet vorzugsweise einen einkernigen gesättigten oder ungesättigten Heterocyclus mit 1 bis 2 N- und/oder O- Atomen, der ein- oder zweifach durch Carbonylsauerstoff, OH oder OA substituiert sein kann.

T bedeutet besonders bevorzugt z.B. 2-Oxo-piperidin-1-yl, 2-Oxo-pyrrolidin-1-yl, 2-Oxo-1*H*-pyridin-1-yl, 3-Oxo-morpholin-4-yl, 4-Oxo-1*H-*pyridin-1-yl, 2,6-Dioxo-piperidinl-yl, 2-Oxo-piperazin-1-yl, 2,6-Dioxo-piperazin-1-yl, 2,5-Dioxo-pyrrolidin-1-yl, 2-Oxo-1,3-oxazolidin-3-yl, 3-Oxo-2*H*-pyridazin-2-yl, 2-Caprolactam-1-yl (= 2-Oxo-azepan-1-yl), 2-Hydroxy-6-oxo-piperazin-1-yl, 2-Methoxy-6-oxo-piperazin-1-yl, 2-Aza-bicyclo[2.2.2]-octan-3-on-2-yl, ganz besonders bevorzugt ist 2-Oxo-piperidin-1-yl.

Die Verbindungen der Formel I gemäß Anspruch 1 können ein oder mehrere chirale Zentren besitzen und daher in verschiedenen stereoisomeren Formen vorkommen. Die Formel I umschließt alle diese Formen.

Die Verbindungen der Formel I gemäß Anspruch 1 und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I gemäß Anspruch 1 umsetzt.

Die Ausgangsverbindungen der Formeln II, III, IV und V sind in der Regel bekannt. Sind sie neu, so können sie aber nach an sich bekannten Methoden hergestellt werden.

Nach folgendem Schema lassen sich alle Verbindungen der folgenden Formel VI (mit R = H oder Methyl; n = 3, 4 oder 5) synthetisieren.

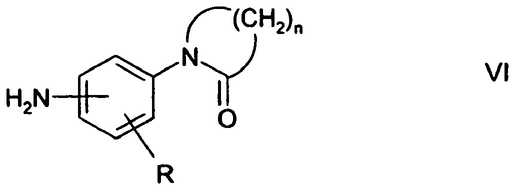

Z.B. Synthese von 1-(4-Amino-2-methylphenyl)-piperidin-2-on:

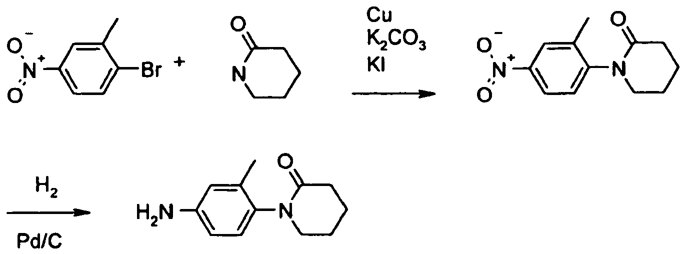

Alternativsynthese:

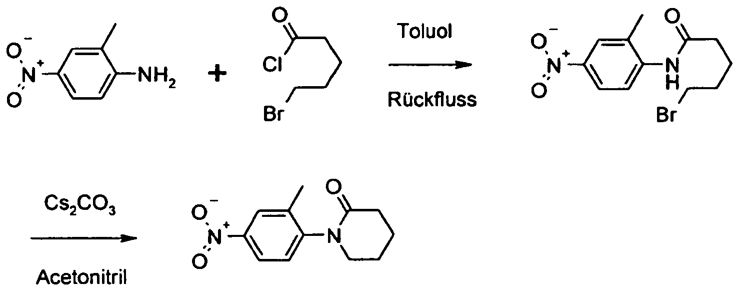

Synthese des Phenylpiperidonbausteins ohne Methylgruppe:

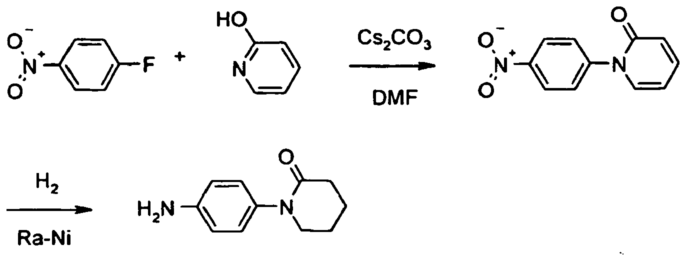

Eine Base der Formel VI kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure.

Verbindungen der Formel I gemäß Anspruch 1 können vorzugsweise erhalten werden, indem man Verbindungen der Formel II mit Verbindungen der Formel III umsetzt.

Die Umsetzung erfolgt in der Regel in einem inerten Lösungsmittel, in Gegenwart eines säurebindenden Mittels vorzugsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums. Auch der Zusatz einer organischen Base wie Triethylamin, Dimethylanilin, Pyridin oder Chinolin oder eines Überschusses der Phenolkomponente der Formel II bzw. des Alkylierungsderivates der Formel III kann günstig sein. Die Reaktionszeit liegt je nach den angewendeten Bedingungen zwischen einigen Minuten und 14 Tagen, die Reaktionstemperatur zwischen etwa 0° und 150°, normalerweise zwischen 20° und 130°.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Verbindungen der Formel I gemäß Anspruch 1 können weiter vorzugsweise erhalten werden, indem man Verbindungen der Formel IV mit Verbindungen der Formel V umsetzt.

Die Umsetzung erfolgt in der Regel in einem inerten Lösungsmittel und unter Bedingungen wie oben angegeben.

In den Verbindungen der Formel IV bedeutet L vorzugsweise Cl, Br, I oder eine freie oder eine reaktionsfähig abgewandelte OH-Gruppe wie z.B. ein aktivierter Ester, ein Imidazolid oder Alkylsulfonyloxy mit 1-6 C-Atomen (bevorzugt Methylsulfonyloxy oder Trifluormethylsulfonyloxy) oder Arylsulfonyloxy mit 6-10 C-Atomen (bevorzugt Phenyl- oder p-Tolylsulfonyloxy).

Derartige Reste zur Aktivierung der Carboxygruppe in typischen Acylierungsreaktionen sind in der Literatur (z.B. in den Standardwerken wie Houben-Weyl, Methoden der organischen Chemie, Georg-Thieme-Verlag, Stuttgart;) beschrieben.

Aktivierte Ester werden zweckmäßig in situ gebildet, z. B. durch Zusatz von HOBt oder N-Hydroxysuccinimid.

Die Umsetzung erfolgt in der Regel in einem inerten Lösungsmittel, in Gegenwart eines säurebindenden Mittels vorzugsweise einer organischen Base wie DIPEA, Triethylamin, Dimethylanilin, Pyridin oder Chinolin oder eines Überschusses der Carboxykomponente der Formel IV.

Auch der Zusatz eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums kann günstig sein.

Die Reaktionszeit liegt je nach den angewendeten Bedingungen zwischen einigen Minuten und 14 Tagen, die Reaktionstemperatur zwischen etwa -30° und 140°, normalerweise zwischen -10° und 90°, insbesondere zwischen etwa 0° und etwa 70°.

Als inerte Lösungsmittel eignen sich die oben genannten.

Verbindungen der Formel I gemäß Anspruch 1 können ferner erhalten werden, indem man Verbindungen der Formel I gemäß Anspruch 1 aus einem ihrer funktionellen Derivate durch Behandeln mit einem solvolysierenden oder hydrogenolysierenden Mittel in Freiheit setzt.

Bevorzugte Ausgangsstoffe für die Solvolyse bzw. Hydrogenolyse sind solche, die sonst der Formel I gemäß Anspruch 1 entsprechen, aber anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, insbesondere solche, die anstelle einer HN-Gruppe eine R'-N-Gruppe tragen, worin R' eine Aminoschutzgruppe bedeutet, und/oder solche, die anstelle des H-Atoms einer Hydroxygruppe eine Hydroxyschutzgruppe tragen, z.B. solche, die der Formel I gemäß Anspruch 1 entsprechen, jedoch anstelle einer Gruppe -COOH eine Gruppe -COOR" tragen, worin R" eine Hydroxyschutzgruppe bedeutet.

Bevorzugte Ausgangsstoffe sind auch die Oxadiazolderivate, die in die entsprechenden Amidinoverbindungen überführt werden können.

Es können auch mehrere - gleiche oder verschiedene - geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls durchgeführt worden ist. Typisch für solche Gruppen sind insbesondere unsubstituierte oder substituierte Acyl-, Aryl-, Aralkoxymethyl- oder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, Ethoxycarbonyl, 2,2,2-Trichlorethoxycarbonyl, BOC (tert.-Butyloxycarbonyl), 2-lodethoxycarbonyl; Aralkyloxycarbonyl wie CBZ ("Carbobenzoxy"), 4-Methoxybenzyloxycarbonyl, FMOC; Arylsulfonyl wie Mtr. Bevorzugte Aminoschutzgruppen sind BOC und Mtr, ferner CBZ, Fmoc, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. Benzyl, 4-Methoxybenzyl, p-Nitrobenzoyl, p-Toluolsulfonyl, tert.-Butyl und Acetyl, wobei Benzyl und tert.-Butyl besonders bevorzugt sind.

Das In-Freiheit-Setzen der Verbindungen der Formel I gemäß Anspruch 1 aus ihren funktionellen Derivaten gelingt - je nach der benutzten Schutzgruppe - z. B. mit starken Säuren, zweckmäßig mit TFA oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Methanol, Ethanol oder Isopropanol, sowie Wasser. Ferner kommen Gemische der vorgenannten Lösungsmittel in Frage. TFA wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9:1. Die Reaktionstemperaturen für die Spaltung liegen zweckmäßig zwischen etwa 0 und etwa 50°, vorzugsweise arbeitet man zwischen 15 und 30° (Raumtemperatur).

Die Gruppen BOC, OBut und Mtr können z. B. bevorzugt mit TFA in Dichlormethan oder mit etwa 3 bis 5n HCl in Dioxan bei 15-30° abgespalten werden, die FMOC-Gruppe mit einer etwa 5- bis 50 %igen Lösung von Dimethylamin, Diethylamin oder Piperidin in DMF bei 15-30°.

Hydrogenolytisch entfernbare Schutzgruppen (z. B. CBZ, Benzyl oder die Freisetzung der Amidinogruppe aus ihrem Oxadiazolderivat)) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z. B. gut an 5 bis 10 %igem Pd/C in Methanol oder mit Ammomiumformiat (anstelle von Wasserstoff) an Pd/C in Methanol/DMF bei 20-30°.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Trifluormethylbenzol, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid, N-Methylpyrrolidon (NMP) oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Ester können z.B. mit Essigsäure oder mit NaOH oder KOH in Wasser, Wasser-THF oder Wasser-Dioxan bei Temperaturen zwischen 0 und 100° verseift werden.

Ferner kann man freie Aminogruppen in üblicher Weise mit einem Säurechlorid oder -anhydrid acylieren oder mit einem unsubstituierten oder substituierten Alkylhalogenid alkylieren, oder mit CH₃-C(=NH)-OEt umsetzen, zweckmäßig in einem inerten Lösungsmittel wie Dichlormethan oder THF und /oder in Gegenwart einer Base wie Triethylamin oder Pyridin bei Temperaturen zwischen -60 und +30°.

Eine Base der Formel I gemäß Anspruch 1 kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Salze mit physiologisch nicht unbedenklichen Säuren, z.B. Pikrate, können zur Isolierung und /oder Aufreinigung der Verbindungen der Formel I gemäß Anspruch 1 verwendet werden.

Andererseits können Verbindungen der Formel I gemäß Anspruch 1 mit Basen (z.B. Natrium- oder Kaliumhydroxid oder -carbonat) in die entsprechenden Metall-, insbesondere Alkalimetall- oder Erdalkalimetall-, oder in die entsprechenden Ammoniumsalze umgewandelt werden.

Auch physiologisch unbedenkliche organische Basen, wie z.B. Ethanolamin können verwendet werden.

Erfindungsgemäße Verbindungen der Formel I gemäß Anspruch 1 können aufgrund ihrer Molekülstruktur chiral sein und können dementsprechend in verschiedenen enantiomeren Formen auftreten. Sie können daher in racemischer oder in optisch aktiver Form vorliegen.

Da sich die pharmazeutische Wirksamkeit der Racemate bzw. der Stereoisomeren der erfindungsgemäßen Verbindungen unterscheiden kann, kann es wünschenswert sein, die Enantiomere zu verwenden. In diesen Fällen kann das Endprodukt oder aber bereits die Zwischenprodukte in enantiomere Verbindungen, durch dem Fachmann bekannte chemische oder physikalische Maßnahmen, aufgetrennt oder bereits als solche bei der Synthese eingesetzt werden.

Im Falle racemischer Amine werden aus dem Gemisch durch Umsetzung mit einem optisch aktiven Trennmittel Diastereomere gebildet. Als Trennmittel eignen sich z.B. optisch aktiven Säuren, wie die R- und S-Formen von Weinsäure, Diacetylweinsäure, Dibenzoylweinsäure, Mandelsäure, Äpfelsäure, Milchsäure, geeignet N-geschützte Aminosäuren (z.B. N-Benzoylprolin oder N-Benzolsulfonylprolin) oder die verschiedenen optisch aktiven Camphersulfonsäuren. Vorteilhaft ist auch eine chromatographische Enantiomerentrennung mit Hilfe eines optisch aktiven Trennmittels (z.B. Dinitrobenzoylphenylglycin, Cellulosetriacetat oder andere Derivate von Kohlenhydraten oder auf Kieselgel fixierte chiral derivatisierte Methacrylatpolymere). Als Laufmittel eignen sich hierfür wäßrige oder alkoholische Lösungsmittelgemische wie z.B. Hexan/Isopropanol/ Acetonitril z.B. im Verhältnis 82:15:3.

Gegenstand der Erfindung sind ferner Arzneimittel, enthaltend mindestens eine Verbindung der Formel I gemäß Anspruch 1 und/oder ihre pharmazeutisch verwendbaren Derivate, Solvate und Stereoisomere, einschließlich deren Mischungen in allen Verhältnissen, sowie gegebenenfalls Träger- und/oder Hilfsstoffe.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder oder auch als Nasenspray. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und /oder mehrere weitere Wirkstoffe enthalten, z.B. ein oder mehrere Vitamine.

Die Verbindungen der Formel I gemäß Anspruch 1 und ihre physiologisch unbedenklichen Salze können bei der Bekämpfung und Verhütung von thromboembolischen Erkrankungen wie Thrombose, myocardialem Infarkt, Arteriosklerose, Entzündungen, Apoplexie, Angina pectoris, Restenose nach Angioplastie, Claudicatio intermittens, Tumoren, Tumorerkrankungen und/oder Tumormetastasen verwendet werden.

Dabei werden die erfindungsgemäßen Substanzen in der Regel vorzugsweise in Dosierungen zwischen etwa 1 und 500 mg, insbesondere zwischen 5 und 100 mg pro Dosierungseinheit verabreicht. Die tägliche Dosierung liegt vorzugsweise zwischen etwa 0,02 und 10 mg/kg Körpergewicht. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel: Ethylacetat/Methanol 9:1.

| | |
|---|---|
| Massenspektrometrie (MS): | EI (Elektronenstoß-Ionisation) M⁺ |
| | FAB (Fast Atom Bombardment) (M+H)⁺ |
| | ESI (Electrospray lonization) (M+H)⁺ (wenn |
| | nichts anderes angegeben) |

### Beispiel 1

### 2-(1-Amino-isochinolin-7-yloxy)-pentansäure-N-[4-(2-oxo-piperidin-1-yl)-phenyl]-amid ("AA"):

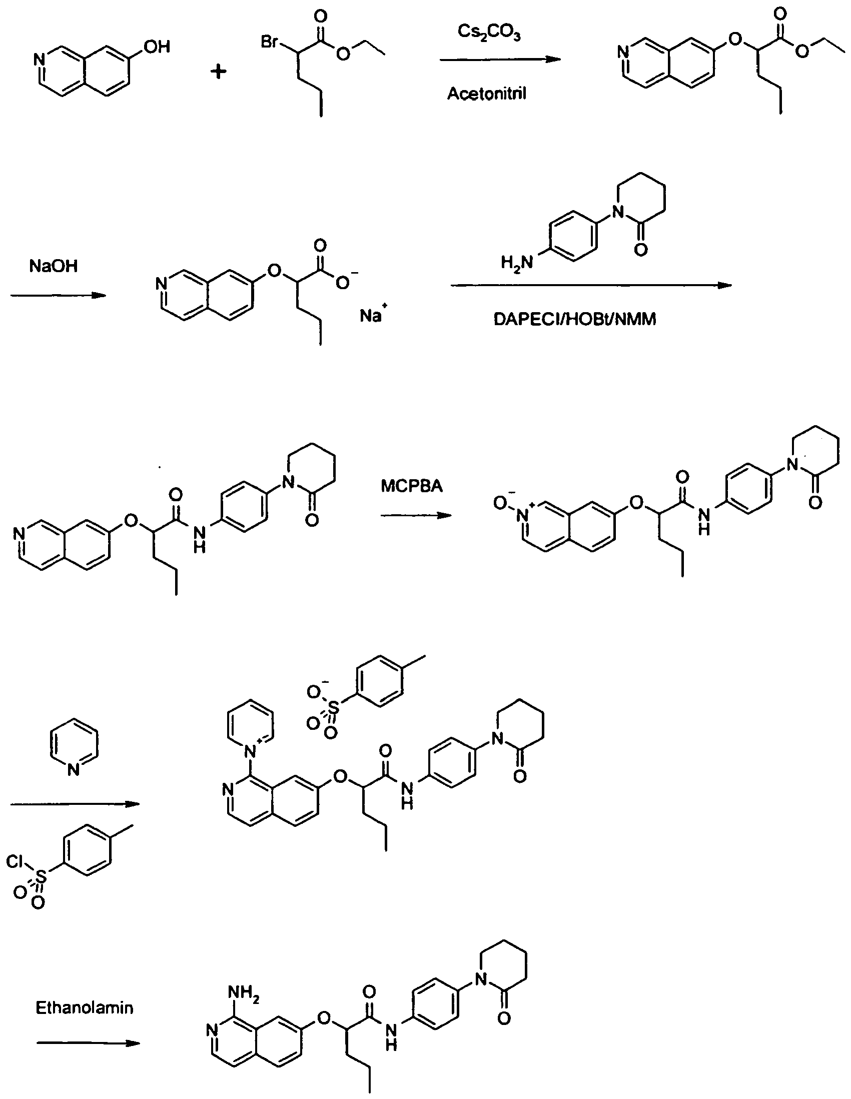

Eine Lösung von 3.00 g (20.7 mmol) 7-Hydroxyisochinolin und 4.33 g (20.7 mmol) 2-Brompentansäure-ethylester in 30 ml Acetonitril wird mit 6.70 g (20.6 mmol) Caesiumcarbonat versetzt und 18 Stunden bei Raumtemperatur gerührt. Das Reaktionsgemisch wird filtriert und das Filtrat eingedampft: 2-(Isochinolin-7-yloxy)-pentansäure-ethylester als farbloser Feststoff; ESI 274.

Eine Lösung von 5.20 g (19.0 mmol) 2-(Isochinolin-7-yloxy)-pentansäure-ethylester in 50 ml Methanol wird mit 10 ml Wasser und 840 mg (21.0 mmol) Natriumhydroxid versetzt und 42 Stunden bei Raumtemperatur gerührt. Das Lösungsmittel wird abdestilliert: 2-(Isochinolin-7-yloxy)-pentansäure Natriumsalz als farbloser Feststoff; ESI 246.

Eine Lösung von 940 mg (3.52 mmol) 2-(Isochinolin-7-yloxy)-pentansäure Natriumsalz, 670 mg (3.52 mmol) 1-(4-Amino-phenyl)-piperidin-2-on, 670 mg (3.52 mmol) N-(3-Dimethylaminopropyl)-N'-ethylcarbodiimidhydrochlorid (DAPECI) und 480 mg (3.56 mmol) 1-Hydroxybenztriazol (HOBt) in 2 ml DMF wird mit 390 ml (3.55 mmol) 4-Methylmorpholin versetzt und 18 Stunden bei Raumtemperatur gerührt. Das Reaktionsgemisch wird auf Wasser gegeben und der Niederschlag abfiltriert: 2-(Isochinolin-7-yloxy)-pentansäure-[4-(2-oxo-piperidin-1-yl)-phenyl]-amid als farbloser Feststoff; ESI 418.

Eine Lösung von 600 mg (1.44 mmol) 2-(Isochinolin-7-yloxy)-pentansäure-[4-(2-oxo-piperidin-1-yl)-phenyl]-amid in 5 ml Aceton wird mit 464 mg (2.69 mmol) 3-Chlorperbenzoesäure versetzt und 18 Stunden bei Raumtemperatur gerührt Das Reaktionsgemisch wird - eingeengt, der Rückstand zwischen Ethylacetat und gesättigter Natriumhydrogencarbonatlösung verteilt. Die organische Phase wird eingedampft: 2-(2-Oxy-isochinolin-7-yloxy)-pentansäure-[4-(2-oxo-piperidin-1-yl)-phenyl]-amid als farbloser Feststoff; ESI 434.

Eine Lösung von 270 mg (0.435 mmol) 2-(2-Oxy-isochinolin-7-yloxy)-pentansäure-[4-(2-oxo-piperidin-1-yl)-phenyl]-amid in 2 ml Pyridin wird mit 137 mg (0.40 mmol) 4-Toluolsulfonylchlorid versetzt und 18 Stunden bei Raumtemperatur gerührt. Das Lösungsmittel wird abdestilliert: 1-(7-{1-[4-(2-Oxo-piperidin-1-yl)-phenylcarbamoyl]-butoxy}-isochinolin-1-yl)-pyridinium Toluol-4-sulfonat als rötlicher Feststoff.

Das so erhaltene rohe 1-(7-{1-[4-(2-Oxo-piperidin-1-yl)-phenylcarbamoyl]-butoxy}-isochinolin-1-yl)-pyridinium Toluol-4-sulfonat wird in 4 ml Ethanolamin gelöst und 42 Stunden bei Raumtemperatur gerührt. Das Reaktionsgemisch wird auf Wasser gegeben und mit Ethylacetat extrahiert. Die organische Phase wird eingedampft und der Rückstand an einer Kieselgelsäule chromatographiert: 2-(1-Amino-isochinolin-7-yloxy)-pentansäure-[4-(2-oxo-piperidin-1-yl)-phenyl]-amid ("AA") als farbloser Feststoff; ESI 433.

Analog erhält man
2-(1-Amino-isochinolin-7-yloxy)-*N*-[4-(2-oxo-piperidin-1-yl)-phenyl]-2-phenyl-acetamid ("AB"), ESI 467;
2-(Isochinolin-7-yloxy)-*N*-[4-(2-oxo-piperidin-1-yl)-phenyl]-2-phenyl-acetamid, ESI 452;
2-(Isochinolin-7-yloxy)-pentansäure-*N*-[4-(2-oxo-piperidin-1-yl)-phenyl]-amid, ESI 418;
2-(1-Amino-isochinolin-7-yloxy)-*N*-[4-(2-oxo-piperidin-1-yl)-phenyl]-acetamid, ESI 391;
2-(Isochinolin-7-yloxy)-*N*-[4-(2-oxo-piperidin-1-yl)-phenyl]-acetamid, ESI 376;
2-(Isochinolin-7-yloxy)-pentansäure-*N*-[4-(2-oxo-pyrrolidin-1-yl)-3-methyl-phenyl]-amid, ESI 418;
2-(Isochinolin-7-yloxy)-pentansäure-*N*-[4-(2-oxo-piperidin-1-yl)-3-methylphenyl]-amid, ESI 432;
2-(Isochinolin-7-yloxy)-pentansäure-*N*-[4-(2-oxo-azepan-1-yl)-3-methylphenyl]-amid, ESI 446;
2-(Isochinolin-7-yloxy)-*N*-[4-(2-oxo-piperidin-1-yl)-3-methyl-phenyl]-acetamid, ESI 390;
2-(Isochinolin-7-yloxy)-*N*-[3-methyl-4-(2-oxo-piperidin-1-yl)-phenyl]-2-(2-fluorphenyl)-acetamid, ESI 484;
2-(Isochinolin-7-yloxy)-*N*-[4-(2-oxo-1*H*-pyrazin-1-yl)-phenyl]-pentansäureamid, ESI 415.

### Beispiel 2

### 2-(2,1,3-Benzothiadiazol-5-ylamino)-N-[4-(2-oxo-piperidin-1-yl)-3-methyl-phenyl]-2-phenyl-acetamid:

Die Herstellung erfolgt analog nachstehendem Reaktionsschema

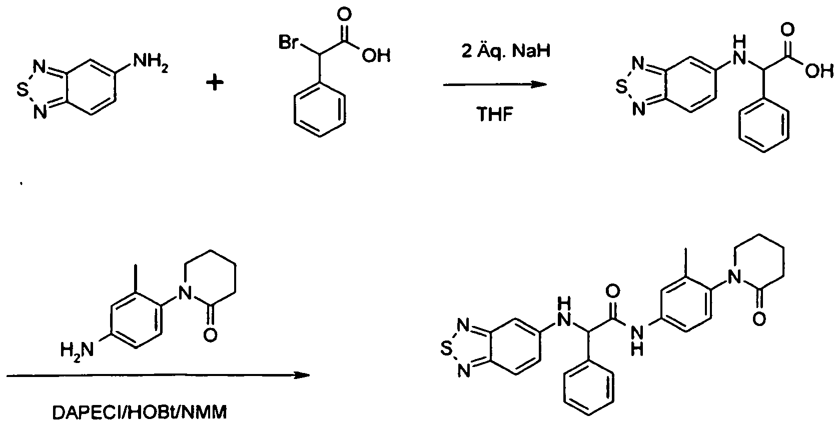

Analog erhält man die nachstehenden Verbindungen
2-(1,3-Benzodioxol-5-ylamino)-*N*-[4-(2-oxo-piperidin-1-yl)-3-methylphenyl]-2-phenyl-acetamid,
2-(Indol-5-ylamino)-*N*-[4-(2-oxo-piperidin-1-yl)-3-methyl-phenyl]-2-phenyl-acetamid,
2-(2-Methyl-benzimidazol-5-ylamino)-*N*-[4-(2-oxo-piperidin-1-yl)-3-methyl-phenyl]-2-phenyl-acetamid,
2-(Benzothiazol-6-ylamino)-*N*-[4-(2-oxo-piperidin-1-yl)-3-methyl-phenyl]-2-phenyl-acetamid,
2-(Isochinolin-7-ylamino)-*N*-[4-(2-oxo-piperidin-1-yl)-3-methyl-phenyl]-2-phenyl-acetamid,
2-(Isochinolin-6-ylamino)-*N*-[4-(2-oxo-piperidin-1-yl)-3-methyl-phenyl]-2-phenyl-acetamid,
2-(1*H*-Indazol-6-ylamino)-*N*-[4-(2-oxo-piperidin-1-yl)-3-methyl-phenyl]-2-phenyl-acetamid,
2-(1*H*-Indazol-5-ylamino)-*N*-[4-(2-oxo-piperidin-1-yl)-3-methyl-phenyl]-2-phenyl-acetamid,
2-(2,3-Dihydro-benzo[1,4]dioxin-6-ylamino)-*N*-[4-(2-oxo-piperidin-1-yl)-3-methyl-phenyl]-2-phenyl-acetamid,
2-(Chinoxalin-6-ylamino)-*N*-[4-(2-oxo-piperidin-1-yl)-3-methyl-phenyl]-2-phenyl-acetamid.

### Pharmakologische Daten

### Affinität zu Rezeptoren

**Tabelle 1**

| Verbindung Nr. | FXa-IC₅₀ [M] | TF/FVIIa-IC₅₀ [M] |
|---|---|---|
| "AA" | 3.5 x 10⁻⁷ | 2.2 x 10⁻⁷ |
| "AB" | 3.5 x 10⁻⁷ | 2.0 x 10⁻⁷ |
| | | |

### Beispiel 3

### Synthese von 1-(4-Amino-2-methylphenyl)-piperidin-2-on:

Ein Gemisch von 5.00 g (23.1 mmol) 2-Brom-5-nitrotoluol, 2.28 g (23.0 mmol) 2-Piperidon, 2.56 g (40 mmol) fein gepulvertes Kupfer, 4.98 g (36.0 mmol) Kaliumcarbonat und 5.98 g (36 mmol) Kaliumiodid) wird 48 Stunden auf 140° C erhitzt. Das Reaktionsgemisch wird auf Wasser gegeben, filtriert und der Rückstand mit Wasser gewaschen. Der Rückstand wird in Ethylacetat aufgenommen und erneut filtriert. Das Filtrat wird getrocknet und eingedampft: 1-(4-Nitro-2-methylphenyl)-piperidin-2-on als gelblicher Feststoff; ESI 235.

Eine Lösung von 4.00 g (17.1 mmol) 1-(4-Nitro-2-methylphenyl)-piperidin-2-on in 150 ml Methanol wird mit 800 mg wasserfeuchtem Palladium auf Aktivkohle versetzt und und 22 Stunden bei Raumtemperatur und Normaldruck hydriert. Das Reaktionsgemisch wird filtriert und das Filtrat eingedampft: 1-(4-Amino-2-methylphenyl)-piperidin-2-on als farbloser Feststoff; ESI 205.

### Beispiel 4

### Analog Beispiel 1 erhält man die nachstehenden Verbindungen

(S)-2-(1-Amino-isochinolin-7-yloxy)-*N*-[4-(3-oxo-morpholin-4-yl)-phenyl]-4-methyl-pentansäureamid, Hydrochlorid, ESI 449;
(S)-2-(1-Amino-isochinolin-7-yloxy)-*N*-[3-methyl-4-(3-oxo-morpholin-4-yl)-phenyl]-4-methyl-pentansäureamid, Hydrochlorid, ESI 463;
(S)-2-(1-Amino-isochinolin-7-yloxy)-*N*-[4-(2-oxo-2*H*-pyridin-1-yl)-phenyl]-4-methyl-pentansäureamid, Hydrochlorid, ESI 449;
2-(1-Amino-isochinolin-7-yloxy)-*N*-[4-(3-oxo-morpholin-4-yl)-phenyl]-pentansäureamid, Hydrochlorid, ESI 435;
2-(1-Amino-isochinolin-7-yloxy)-*N*-[4-(3-oxo-morpholin-4-yl)-phenyl]-2-(2-chlorphenyl)-acetamid,
2-(1-Amino-isochinolin-7-yloxy)-*N*-[4-(3-oxo-2*H*-pyridazin-2-yl)-phenyl]-4-methyl-pentansäureamid,
2-(1-Amino-isochinolin-7-yloxy)-*N*-[4-(2-oxo-1*H*-pyrazin-1-yl)-phenyl]-4-methyl-pentansäureamid,
2-(1-Amino-isochinolin-7-yloxy)-*N*-[4-(2-oxo-piperazin-1-yl)-phenyl]-4-methyl-pentansäureamid,
2-(1-Amino-isochinolin-7-yloxy)-*N*-[4-(2-oxo-[1,3]oxazinan-3-yl)-phenyl]-4-methyl-pentansäureamid,
2-(1 -Amino-isochinolin-7-yloxy)-*N*-[4-(2-oxo-3*H*-thiazol-3-yl)-phenyl]-4-methyl-pentansäureamid,
2-(1-Amino-isochinolin-7-yloxy)-*N*-[4-(2-oxo-oxazolidin-3-yl)-phenyl]-4-methyl-pentansäureamid,
2-(1-Amino-isochinolin-7-yloxy)-*N*-[3-methyl-4-(2-oxo-oxazolidin-3-yl)-phenyl]-4-methyl-pentansäureamid,
2-(1-Amino-isochinolin-7-yloxy)-*N*-[4-(2-oxo-tetrahydro-pyrimidin-1-yl)-phenyl]-4-methyl-pentansäureamid,
2-(1-Amino-isochinolin-7-yloxy)-*N*-[4-(2-oxo-imidazolidin-1-yl)-phenyl]-4-methyl-pentansäureamid,
2-(1-Amino-isochinolin-7-yloxy)-*N*-[3-methyl-4-(2-oxo-azepan-1-yl)-phenyl]-pentansäureamid, Hydrochlorid, ESI 461;
2-(1-Amino-isochinolin-7-yloxy)-*N*-[3-methyl-4-(2-oxo-piperidin-1-yl)-phenyl]-pentansäureamid, Hydrochlorid, ESI 447;
2-(1-Amino-isochinolin-7-yloxy)-*N*-[3-methyl-4-(2-oxo-piperidin-1-yl)-phenyl]-acetamid, Hydrochlorid, ESI 405;
2-(1-Amino-isochinolin-7-yloxy)-*N*-[3-trifluormethyl-4-(2-Aza-bicyclo[2.2.2]-octan-3-on-2-yl)-phenyl]-pentansäureamid, ESI 527;
2-(1-Amino-isochinolin-7-yloxy)-*N*-[4-(2-oxo-piperidin-1-yl)-phenyl]-propionamid, Hydrochlorid, ESI 405;
2-(1-Amino-isochinolin-7-yloxy)-*N*-[4-(2-oxo-piperidin-1-yl)-phenyl]-buttersäureamid, Hydrochlorid, ESI 419;
2-(1-Amino-isochinolin-7-yloxy)-*N*-[3-methyl-4-(2-oxo-pyrrolidin-1-yl)-phenyl]-buttersäureamid, Hydrochlorid, ESI 419;
2-(1-Amino-isochinolin-7-yloxy)-*N*-[4-(2-oxo-piperidin-1-yl)-phenyl]-pentansäureamid, Hydrochlorid, ESI 433;
2-(1-Amino-isochinolin-7-yloxy)-*N*-[4-(2-oxo-pyrrolidin-1-yl)-phenyl]-hexansäureamid, Hydrochlorid, ESI 433;
2-(1-Amino-isochinolin-7-yloxy)-*N*-[3-methyl-4-(2-oxo-piperidin-1-yl)-phenyl]-hexansäureamid, Hydrochlorid, ESI 461;
2-(1-Amino-isochinolin-7-yloxy)-*N*-[4-(2-oxo-2*H*-pyridin-1-yl)-phenyl]-buttersäureamid, Hydrochlorid, ESI 415;
2-(1-Amino-isochinolin-7-yloxy)-*N*-[4-(2-oxo-2*H*-pyridin-1-yl)-phenyl]-pentansäureamid, Hydrochlorid, ESI 429;
2-(1-Amino-isochinolin-7-yloxy)-*N*-[3-chlor-4-(2-oxo-pyrrolidin-1-yl)-phenyl]-pentansäureamid, Hydrochlorid, ESI 453;
2-(1-Amino-isochinolin-7-yloxy)-*N*-[3-methyl-4-(2-oxo-piperidin-1-yl)-phenyl]-buttersäureamid, Hydrochlorid, ESI 433;
2-(1-Amino-isochinolin-7-yloxy)-*N*-[4-(2-oxo-piperidin-1-yl)-phenyl]-hexansäureamid, Hydrochlorid, ESI 447;
2-(1-Amino-isochinolin-7-yloxy)-*N*-[4-(2-oxo-piperidin-1-yl)-phenyl]-4-methylpentansäureamid, Hydrochlorid, ESI 447;
2-(1-Amino-isochinolin-7-yloxy)-*N*-[3-methyl-4-(2-oxo-piperidin-1-yl)-phenyl]-4-methylpentansäureamid, Hydrochlorid, ESI 461;
2-(1-Amino-isochinolin-7-yloxy)-*N*-[4-(2-oxo-2*H*-pyridin-1-yl)-phenyl]-4-methyl-pentansäureamid, Hydrochlorid, ESI 443;
2-(1-Amino-isochinolin-7-yloxy)-*N*-[4-(2-oxo-piperidin-1-yl)-phenyl]-2-(2-fluorphenyl)-acetamid, Hydrochlorid, ESI 485;
2-(1-Amino-isochinolin-7-yloxy)-*N*-[3-rnethyl-4-(2-oxo-piperidin-1-yl)-phenyl]-2-(2-fluorphenyl)-acetamid, Hydrochlorid, ESI 499;
(R)-2-(1-Amino-isochinolin-7-yloxy)-*N*-[4-(2-oxo-2*H*-pyridin-1-yl)-phenyl]-4-methyl-pentansäureamid, Hydrochlorid, ESI 443;
2-(1-Amino-isochinolin-7-yloxy)-*N*-[4-(2-oxo-2*H*-pyridin-1-yl)-phenyl]-4,4-dimethyl-pentansäureamid, Hydrochlorid, ESI 457;
2-(1-Amino-isochinolin-7-yloxy)-*N*-[4-(2-oxo-2*H*-pyridin-1-yl)-phenyl]-2-(2-fluorphenyl)-acetamid, Hydrochlorid, ESI 481;
2-(1-Amino-isochinolin-7-yloxy)-*N*-[4-(3-oxo-morpholin-4-yl)-phenyl]-buttersäureamid, Hydrochlorid, ESI 421;
2-(1-Amino-isochinolin-7-yloxy)-*N*-[3-methyl-4-(3-oxo-morpholin-4-yl)-phenyl]-buttersäureamid, Hydrochlorid, ESI 435;
2-(1-Amino-isochinolin-7-yloxy)-*N*-[3-methyl-4-(3-oxo-morpholin-4-yl)-phenyl]-pentansäureamid, Hydrochlorid, ESI 449;
2-(1-Amino-isochinolin-7-yloxy)-*N*-[3-chlor-4-(2-oxo-2*H*-pyridin-1-yl)-phenyl]-4-methyl-pentansäureamid, Hydrochlorid, ESI 477;
2-(1-Amino-isochinolin-7-yloxy)-*N*-[4-(3-oxo-morpholin-4-yl)-phenyl]-2-(2-fluorphenyl)-acetamid, Hydrochlorid, ESI 487;
2-(1-Amino-isochinolin-7-yloxy)-*N*-[3-methyl-4-(3-oxo-morpholin-4-yl)-phenyl]-4-methyl-pentansäureamid, Hydrochlorid, ESI 463;
2-(1-Amino-isochinolin-7-yloxy)-*N*-[3-chlor-4-(2-oxo-pyrrolidin-1-yl)-phenyl]-2-(2-fluorphenyl)-acetamid, Hydrochlorid, ESI 505;
2-(1-Amino-isochinolin-7-yloxy)-*N*-[3-chlor-4-(2-oxo-2*H*-pyridin-1-yl)-phenyl]-buttersäureamid, ESI 449;
2-(1-Amino-isochinolin-7-yloxy)-*N*-[2-fluor-4-(2-oxo-2*H*-pyridin-1-yl)-phenyl]-pentansäureamid, ESI 447;
2-(1-Amino-isochinolin-7-yloxy)-*N*-[2-methyl-4-(2-oxo-2*H*-pyridin-1-yl)-phenyl]-4-methyl-pentansäureamid, ESI 457;
2-(1-Amino-isochinolin-7-yloxy)-*N*-[3-chlor-4-(2-oxo-2*H*-pyridin-1-yl)-phenyl]-pentansäureamid, ESI 463;
2-(1-Amino-isochinolin-7-yloxy)-*N*-[4-(3-oxo-morpholin-4-yl)-phenyl]-4-methyl-pentansäureamid, Hydrochlorid, ESI 449;
2-(1-Amino-isochinolin-7-yloxy)-*N*-[3-trifluormethyl-4-(3-oxo-morpholin-4-yl)-phenyl]-4-methyl-pentansäureamid, Hydrochlorid, ESI 517;
2-(1-Amino-isochinolin-7-yloxy)-*N*-[3-trifluormethyl-4-(3-oxo-morpholin-4-yl)-phenyl]-pentansäureamid, Hydrochlorid, ESI 503.

### Beispiel 5

### 2-(1-Amino-isochinolin-7-ylamino)-N-[4-(2-oxo-2H-pyridin-1-yl)-phenyl]-4-methyl-pentansäureamid, Hydrochlorid, ESI 442

Die Herstellung erfolgt analog nachstehendem Reaktionsschema

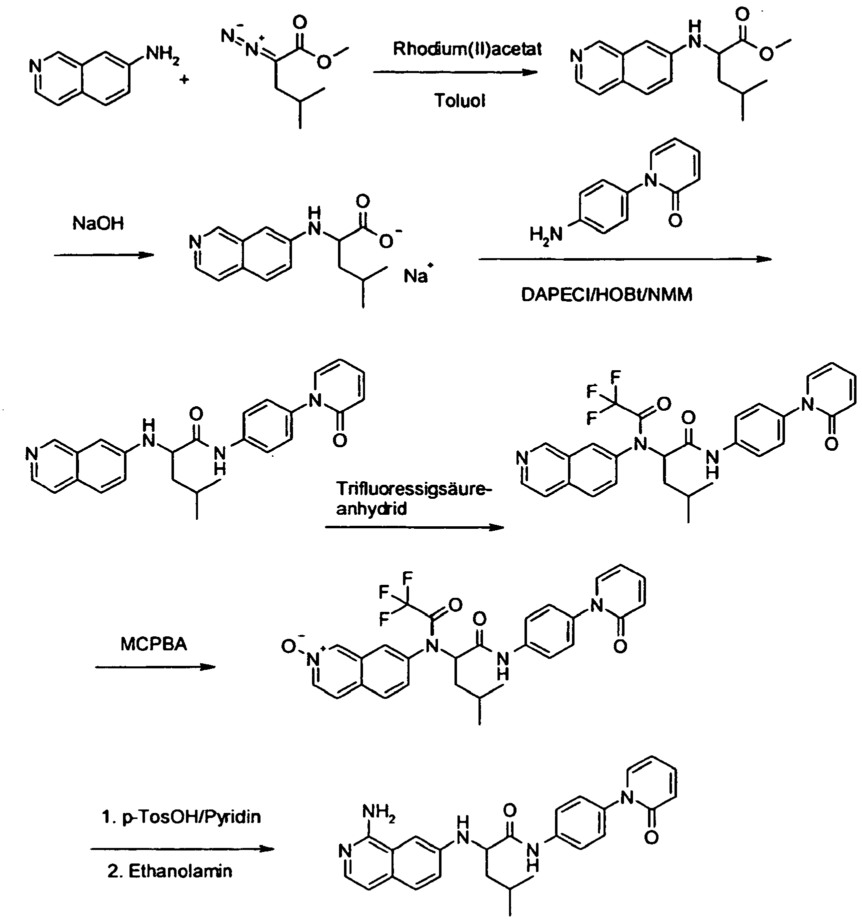

### 1. Stufe:

Eine Lösung von 5.19 g (36.0 mmol) 7-Aminoisochinolin und 5.47 g (35.0 mmol) 2-Diazo-4-methylpentansäuremethylester in 200 ml Toluol wird mit 800 mg Rhodium(II)acetat (dimer) versetzt und 12 Stunden auf 80° C erhitzt. Das abgekühlte Reaktionsgemisch wird filtriert und das Filtrat eingedampft. Der Rückstand wird an einer Kieselgelsäule mit Ethylacetat als Laufmittel chromatographiert: 2-(Isochinolin-7-ylamino)-4-methyl-pentansäuremethylester als gelblicher Feststoff; ESI 273.

### 3. Stufe:

Eine Lösung von 200 mg (469 mmol) 2-(Isochinolin-7-ylamino)-4-methyl-pentansäure-[4-(2-oxo-2*H*-pyridin-1-yl)-phenyl]-amid in 5 ml Dichlormethan wird mit 1 ml Trifluoressigsäureanhydrid versetzt und 18 Stunden bei Raumtemperatur gerührt. Das Reaktionsgemisch wird zwischen Wasser und Dichlormethan verteilt. Die organische Phase wird eingedampft: 2-[Isochinolin-7-yl-trifluoracetylamino]-4-methylpentansäure-[4-(2-oxo-2*H*-pyridin-1-yl)-phenyl]-amid als farbloser Feststoff; ESI 523.

Die anderen Stufen werden analog Beispiel 1 oder nach allgemeinen bekannten Vorschriften hergestellt.

### Analog erhält man die nachstehende Verbindung

(S)-2-(1-Amino-isochinolin-7-ylamino)-*N*-[4-(3-oxo-morpholin-4-yl)-phenyl]-4-methyl-pentansäureamid, Hydrochlorid, ESI 448.

### Beispiel 6

### 2-(8-Oxo-5,6,7,8-tetrahydro-naphthalin-2-yloxy)-N-[3-methyl-4-(2-oxo-piperidin-1-yl)-phenyl]-2-phenyl-acetamid und 2-(8-Amino-5,6,7,8-tetrahydro-naphthalin-2-yloxy)-N-[3-methyl-4-(2-oxo-piperidin-1-yl)-phenyl]-2-phenyl-acetamid, Hydrochlorid, ESI 484

Die Herstellung erfolgt analog nachstehendem Reaktionsschema

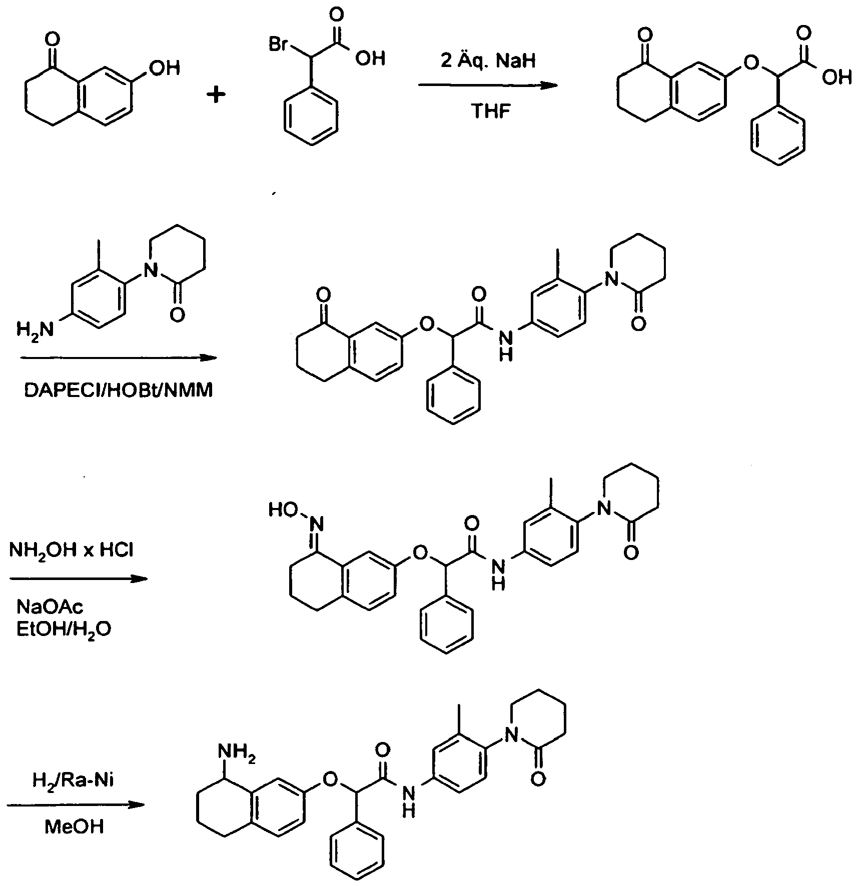

### Analog erhält man

2-(8-Oxo-5,6,7,8-tetrahydro-naphthalin-2-yloxy)-*N*-[3-methyl-4-(2-oxo-piperidin-1-yl)-phenyl]-2-(2-fluorphenyl)-acetamid, ESI 501.

### Beispiel 7

### 2-(Chinolin-6-ylamino)-N-[4-(2-oxo-2H-pyridin-1-yl)-phenyl]-2-phenyl-acetamid, ESI 447

Die Herstellung erfolgt analog nachstehendem Reaktionsschema

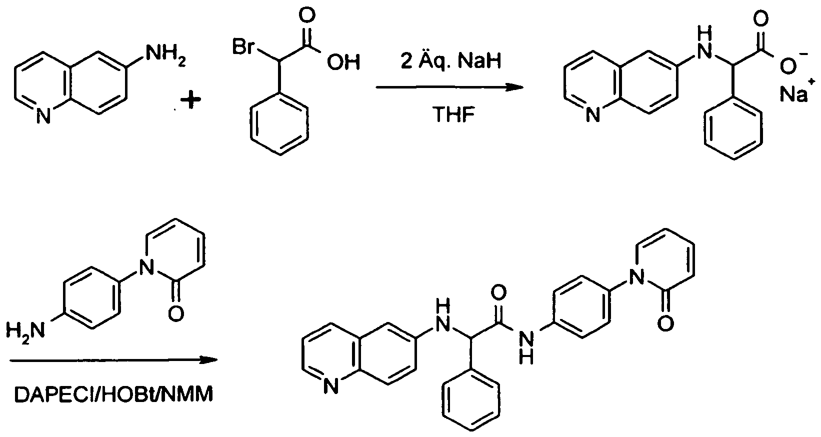

Analog erhält man
2-(Chinolin-6-ylamino)-*N*-[4-(2-oxo-piperidin-1-yl)-phenyl]-2-phenyl-acetamid, ESI 451;
2-(Chinolin-6-ylamino)-*N*-[3-methyl-4-(2-oxo-piperidin-1-yl)-phenyl]-2-phenyl-acetamid, ESI 465;
2-(Isochinolin-7-ylamino)-*N*-[4-(2-oxo-2*H*-pyridin-1-yl)-phenyl]-4-methyl-pentansäureamid, Hydrochlorid, ESI 427.

### Beispiel 8

### 2-(3-Amino-1H-indazol-5-yloxy)-N-[4-(3-oxo-morpholin-4-yl)-phenyl]-4-methyl-pentansäureamid

Die Herstellung erfolgt analog nachstehendem Reaktionsschema

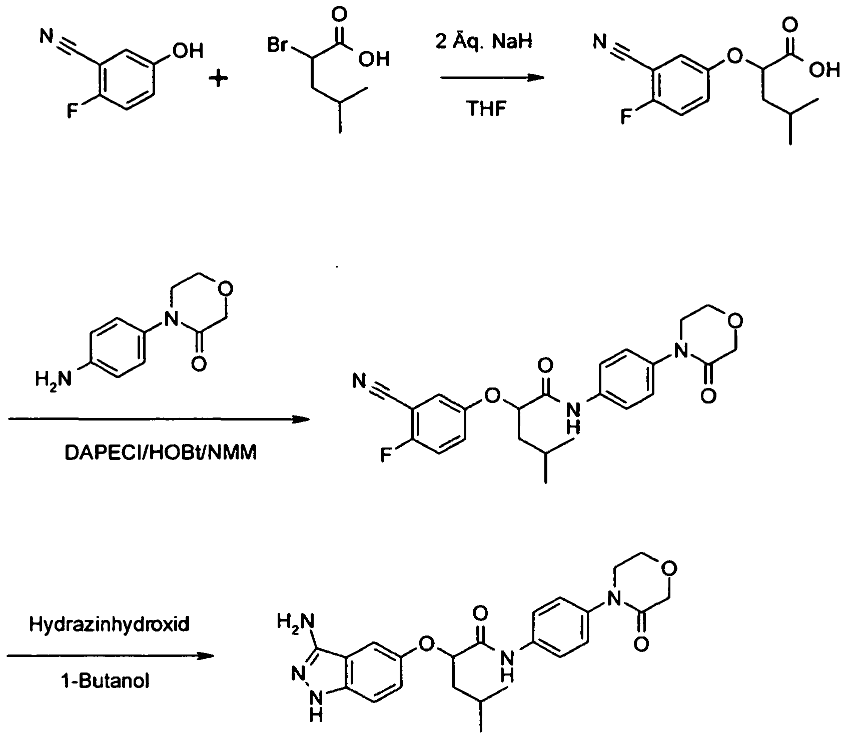

### Beispiel 9

### 2-(3-Amino-benzo[d]isoxazol-5-yloxy)-N-[4-(3-oxo-morpholin-4-yl)-phenyl]-4-methyl-pentansäureamid

Die Herstellung erfolgt analog nachstehendem Reaktionsschema

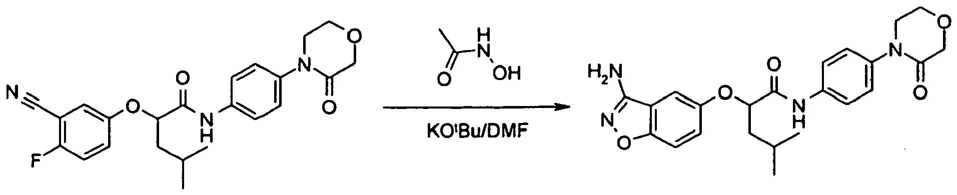

### Beispiel 10

### 2-(3-Amino-benzo[d]isothiazol-5-ylamino)-N-[4-(3-oxo-morpholin-4-yl)-phenyl]-2-(2-fluorphenyl)-acetamid

Die Herstellung erfolgt analog nachstehendem Reaktionsschema

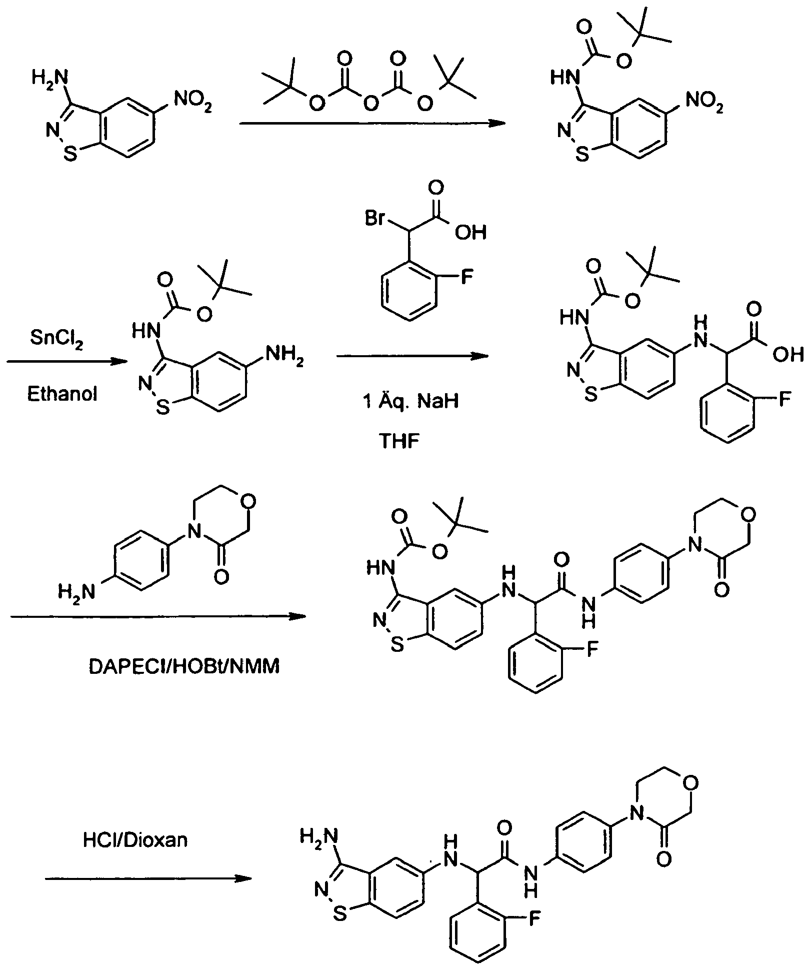

### Beispiel 11

### 2-(3-Amino-benzo[c]isothiazol-5-yloxy)-N-[4-(3-oxo-morpholin-4-yl)-phenyl]-4-methyl-pentansäureamid

Die Herstellung erfolgt analog nachstehendem Reaktionsschema

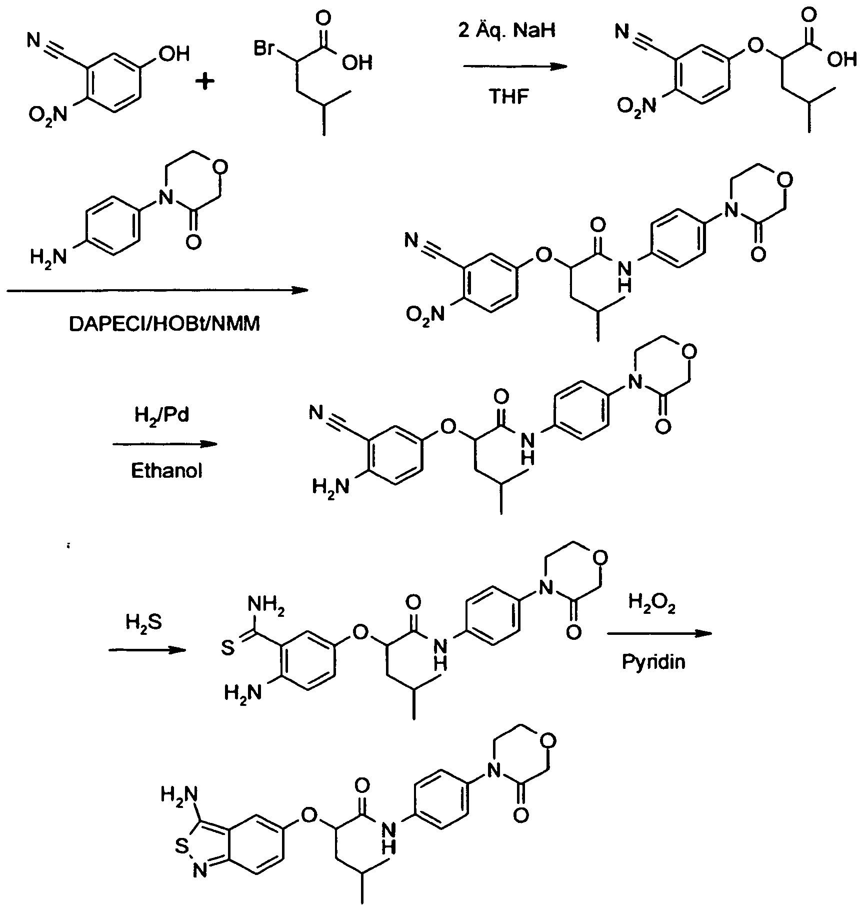

Analog erhält man
2-(3-Amino-benzo[*d*]isothiazol-5-yloxy)-*N*-[4-(3-oxo-morpholin-4-yl)-phenyl]-4-methyl-pentansäureamid.

### Beispiel 12

### 2-(3-Amino-1H-indazol-5-ylamino)-N-[4-(3-oxo-morpholin-4-yl)-phenyl]-4-methyl-pentansäureamid

Die Herstellung erfolgt analog nachstehendem Reaktionsschema

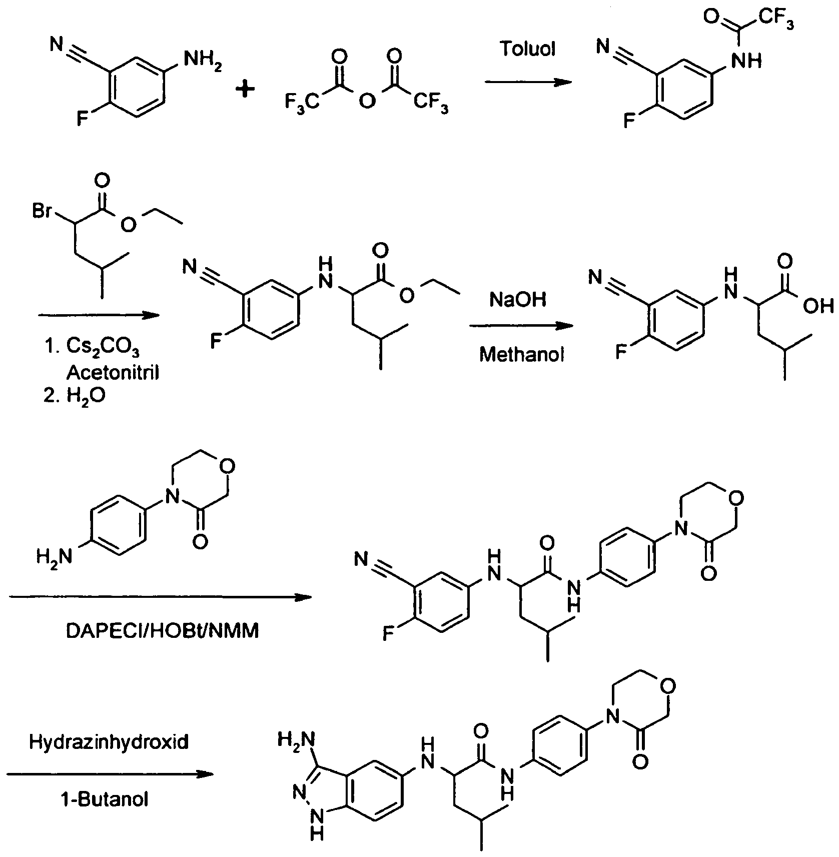

### Beispiel 13

### 2-(3-Amino-benzo[d]isoxazol-5-ylamino)-N-[4-(3-oxo-morpholin-4-yl)-phenyl]-4-methyl-pentansäureamid

Die Herstellung erfolgt analog nachstehendem Reaktionsschema

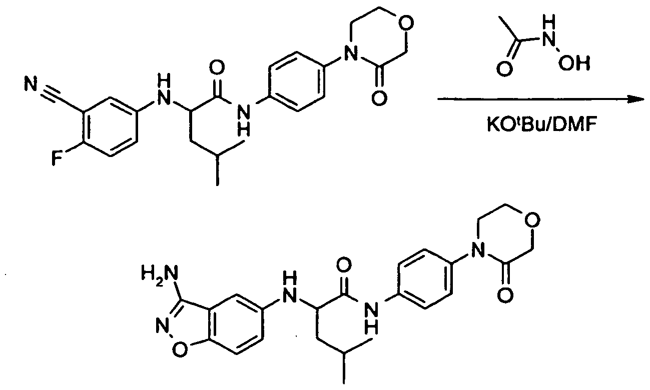

Die nachfolgenden Beispiele betreffen pharmazeutische Zubereitungen:

### Beispiel A: Injektionsgläser

Eine Lösung von 100 g eines Wirkstoffes der Formel I gemäß Anspruch 1 und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I gemäß Anspruch 1 mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I gemäß Anspruch 1, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines Wirkstoffes der Formel I gemäß Anspruch 1 mit 99,5 g Vaseline unter aseptischen Bedingungen.

### Beispiel E: Tabletten

Ein Gemisch von 1 kg Wirkstoff der Formel I gemäß Anspruch 1, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I gemäß Anspruch 1 werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I gemäß Anspruch 1 in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen ausgewählt aus der Gruppe
2-(Isochinolin-7-yloxy)-*N*-[4-(2-oxo-piperidin-1-yl)-phenyl]-2-phenyl-acetamid,
2-(1-Amino-isochinolin-7-yloxy)-*N*-[4-(2-oxo-piperidin-1-yl)-phenyl]-2-phenyl-acetamid,
2-(Isochinolin-7-yloxy)-*N*-[4-(2-oxo-piperidin-1-yl)-phenyl]-2-phenyl-acetamid,
2-(Isochinolin-7-yloxy)-pentansäure-*N*-[4-(2-oxo-piperidin-1-yl)-phenyl]-amid,
2-(1-Amino-isochinolin-7-yloxy)-*N*-[4-(2-oxo-piperidin-1-yl)-phenyl]-acetamid,
2-(Isochinolin-7-yloxy)-*N*-[4-(2-oxo-piperidin-1-yl)-phenyl]-acetamid,
2-(Isochinolin-7-yloxy)-pentansäure-*N*-[4-(2-oxo-pyrrolidin-1-yl)-3-methyl-phenyl]-amid,
2-(Isochinolin-7-yloxy)-pentansäure-*N*-[4-(2-oxo-piperidin-1-yl)-3-methyl-phenyl]-amid,
2-(Isochinolin-7-yloxy)-pentansäure-*N*-[4-(2-oxo-azepan-1-yl)-3-methyl-phenyl]-amid,
2-(Isochinolin-7-yloxy)-*N*-[4-(2-oxo-piperidin-1-yl)-3-methylphenyl]-acetamid,
2-(2,1,3-Benzothiadiazol-5-ylamino)-*N*-[4-(2-oxo-piperidin-1-yl)-3-methyl-phenyl]-2-phenyl-acetamid,
2-(1,3-Benzodioxol-5-ylamino)-*N*-[4-(2-oxo-piperidin-1-yl)-3-methyl-phenyl]-2-phenyl-acetamid,
2-(Indol-5-ylamino)-*N*-[4-(2-oxo-piperidin-1-yl)-3-methylphenyl]-2-phenyl-acetamid,
2-(2-Methyl-benzimidazol-5-ylamino)-*N*-[4-(2-oxo-piperidin-1-yl)-3-methyl-phenyl]-2-phenyl-acetamid,
2-(Benzothiazol-6-ylamino)-*N*-[4-(2-oxo-piperidin-1-yl)-3-methyl-phenyl]-2-phenyl-acetamid,
2-(Isochinolin-7-ylamino)-*N*-[4-(2-oxo-piperidin-1-yl)-3-methylphenyl]-2-phenyl-acetamid,
2-(Isochinolin-6-ylamino)-*N*-[4-(2-oxo-piperidin-1-yl)-3-methylphenyl]-2-phenyl-acetamid,
2-(1*H*-Indazol-6-ylamino)-*N*-[4-(2-oxo-piperidin-1-yl)-3-methylphenyl]-2-phenyl-acetamid,
2-(1*H*-Indazol-5-ylamino)-*N*-[4-(2-oxo-piperidin-1-yl)-3-methylphenyl]-2-phenyl-acetamid,
2-(2,3-Dihydro-benzo[1,4]dioxin-6-ylamino)-*N*-[4-(2-oxo-piperidin-1-yl)-3-methyl-phenyl]-2-phenyl-acetamid,
2-(Chinoxalin-6-ylamino)-*N*-[4-(2-oxo-piperidin-1-yl)-3-methylphenyl]-2-phenyl-acetamid,
2-(Isochinolin-7-yloxy)-*N*-[3-methyl-4-(2-oxo-piperidin-1-yl)-phenyl]-2-(2-fluorphenyl)-acetamid,
2-(Isochinolin-7-yloxy)-*N*-[4-(2-oxo-1*H*-pyrazin-1-yl)-phenyl]-pentansäureamid,
(S)-2-(1-Amino-isochinolin-7-yloxy)-*N*-[4-(3-oxo-morpholin-4-yl)-phenyl]-4-methyl-pentansäureamid,
(S)-2-(1-Amino-isochinolin-7-yloxy)-*N*-[3-methyl-4-(3-oxo-morpholin-4-yl)-phenyl]-4-methyl-pentansäureamid,
(S)-2-(1-Amino-isochinolin-7-yloxy)-*N*-[4-(2-oxo-2*H*-pyridin-1-yl)-phenyl]-4-methyl-pentansäureamid,
2-(1-Amino-isochinolin-7-yloxy)-*N*-[4-(3-oxo-morpholin-4-yl)-phenyl]-pentansäureamid,
2-(1-Amino-isochinolin-7-yloxy)-*N*-[4-(3-oxo-morpholin-4-yl)-phenyl]-2-(2-chlorphenyl)-acetamid,
2-(1-Amino-isochinolin-7-yloxy)-*N*-[4-(3-oxo-2*H*-pyridazin-2-yl)-phenyl]-4-methyl-pentansäureamid,
2-(1-Amino-isochinolin-7-yloxy)-*N*-[4-(2-oxo-1*H*-pyrazin-1-yl)-phenyl]-4-methyl-pentansäureamid,
2-(1-Amino-isochinolin-7-yloxy)-*N*-[4-(2-oxo-piperazin-1-yl)-phenyl]-4-methyl-pentansäureamid,
2-(1-Amino-isochinolin-7-yloxy)-*N*-[4-(2-oxo-[1,3]oxazinan-3-yl)-phenyl]-4-methyl-pentansäureamid,
2-(1-Amino-isochinolin-7-yloxy)-*N*-[4-(2-oxo-3*H*-thiazol-3-yl)-phenyl]-4-methyl-pentansäureamid,
2-(1-Amino-isochinolin-7-yloxy)-*N*-[4-(2-oxo-oxazolidin-3-yl)-phenyl]-4-methyl-pentansäureamid,
2-(1-Amino-isochinolin-7-yloxy)-*N*-[3-methyl-4-(2-oxo-oxazolidin-3-yl)-phenyl]-4-methyl-pentansäureamid,
2-(1-Amino-isochinolin-7-yloxy)-*N*-[4-(2-oxo-tetrahydro-pyrimidin-1-yl)-phenyl]-4-methyl-pentansäureamid,
2-(1-Amino-isochinolin-7-yloxy)-*N*-[4-(2-oxo-imidazolidin-1-yl)-phenyl]-4-methyl-pentansäureamid,
2-(1-Amino-isochinolin-7-yloxy)-*N*-[3-methyl-4-(2-oxo-azepan-1-yl)-phenyl]-pentansäureamid,
2-(1-Amino-isochinolin-7-yloxy)-*N*-[3-methyl-4-(2-oxo-piperidin-1-yl)-phenyl]-pentansäureamid,
2-(1-Amino-isochinolin-7-yloxy)-*N*-[3-methyl-4-(2-oxo-piperidin-1-yl)-phenyl]-acetamid,
2-(1-Amino-isochinolin-7-yloxy)-*N*-[3-trifluormethyl-4-(2-Aza-bicyclo[2.2.2]-octan-3-on-2-yl)-phenyl]-pentansäureamid,
2-(1-Amino-isochinolin-7-yloxy)-*N*-[4-(2-oxo-piperidin-1-yl)-phenyl]-propionamid,
2-(1-Amino-isochinolin-7-yloxy)-*N*-[4-(2-oxo-piperidin-1-yl)-phenyl]-buttersäureamid,
2-(1-Amino-isochinolin-7-yloxy)-*N*-[3-methyl-4-(2-oxo-pyrrolidin-1-yl)-phenyl]-buttersäureamid,
2-(1-Amino-isochinolin-7-yloxy)-*N*-[4-(2-oxo-piperidin-1-yl)-phenyl]-pentansäureamid,
2-(1-Amino-isochinolin-7-yloxy)-*N*-[4-(2-oxo-pyrrolidin-1-yl)-phenyl]-hexansäureamid,
2-(1-Amino-isochinolin-7-yloxy)-*N*-[3-methyl-4-(2-oxo-piperidin-1-yl)-phenyl]-hexansäureamid,
2-(1-Amino-isochinolin-7-yloxy)-*N*-[4-(2-oxo-2*H*-pyridin-1-yl)-phenyl]-buttersäureamid,
2-(1-Amino-isochinolin-7-yloxy)-*N*-[4-(2-oxo-2*H*-pyridin-1-yl)-phenyl]-pentansäureamid,
2-(1-Amino-isochinolin-7-yloxy)-*N*-[3-chlor-4-(2-oxo-pyrrolidin-1-yl)-phenyl]-pentansäureamid,
2-(1-Amino-isochinolin-7-yloxy)-*N*-[3-methyl-4-(2-oxo-piperidin-1-yl)-phenyl]-buttersäureamid,
2-(1-Amino-isochinolin-7-yloxy)-*N*-[4-(2-oxo-piperidin-1-yl)-phenyl]-hexansäureamid,
2-(1-Amino-isochinolin-7-yloxy)-*N*-[4-(2-oxo-piperidin-1-yl)-phenyl]-4-methylpentansäureamid,
2-(1-Amino-isochinolin-7-yloxy)-*N*-[3-methyl-4-(2-oxo-piperidin-1-yl)-phenyl]-4-methylpentansäureamid, Hydrochlorid, ESI 461;
2-(1-Amino-isochinolin-7-yloxy)-*N*-[4-(2-oxo-2*H*-pyridin-1-yl)-phenyl]-4-methyl-pentansäureamid,
2-(1-Amino-isochinolin-7-yloxy)-*N*-[4-(2-oxo-piperidin-1-yl)-phenyl]-2-(2-fluorphenyl)-acetamid,
2-(1-Amino-isochinolin-7-yloxy)-*N*-[3-methyl-4-(2-oxo-piperidin-1-yl)-phenyl]-2-(2-fluorphenyl)-acetamid,
(R)-2-(1-Amino-isochinolin-7-yloxy)-*N*-[4-(2-oxo-2*H*-pyridin-1-yl)-phenyl]-4-methyl-pentansäureamid,
2-(1-Amino-isochinolin-7-yloxy)-*N*-[4-(2-oxo-2*H*-pyridin-1-yl)-phenyl]-4,4-dimethyl-pentansäureamid,
2-(1-Amino-isochinolin-7-yloxy)-*N*-[4-(2-oxo-2*H*-pyridin-1-yl)-phenyl]-2-(2-fluorphenyl)-acetamid,
2-(1-Amino-isochinolin-7-yloxy)-*N*-[4-(3-oxo-morpholin-4-yl)-phenyl]-buttersäureamid,
2-(1-Amino-isochinolin-7-yloxy)-*N*-[3-methyl-4-(3-oxo-morpholin-4-yl)-phenyl]-buttersäureamid,
2-(1-Amino-isochinolin-7-yloxy)-*N*-[3-methyl-4-(3-oxo-morpholin-4-yl)-phenyl]-pentansäureamid,
2-(1-Amino-isochinolin-7-yloxy)-*N*-[3-chlor-4-(2-oxo-2*H-*pyridin-1-yl)-phenyl]-4-methyl-pentansäureamid,
2-(1-Amino-isochinolin-7-yloxy)-*N*-[4-(3-oxo-morpholin-4-yl)-phenyl]-2-(2-fluorphenyl)-acetamid,
2-(1-Amino-isochinolin-7-yloxy)-*N*-[3-methyl-4-(3-oxo-morpholin-4-yl)-phenyl]-4-methyl-pentansäureamid,
2-(1-Amino-isochinolin-7-yloxy)-*N*-[3-chlor-4-(2-oxo-pyrrolidin-1-yl)-phenyl]-2-(2-fluorphenyl)-acetamid,
2-(1-Amino-isochinolin-7-yloxy)-*N*-[3-chlor-4-(2-oxo-2*H-*pyridin-1-yl)-phenyl]-buttersäureamid,
2-(1-Amino-isochinolin-7-yloxy)-*N*-[2-fluor-4-(2-oxo-2*H-*pyridin-1-yl)-phenyl]-pentansäureamid,
2-(1-Amino-isochinolin-7-yloxy)-*N*-[2-methyl-4-(2-oxo-2*H-*pyridin-1-yl)-phenyl]-4-methyl-pentansäureamid,
2-(1-Amino-isochinolin-7-yloxy)-*N*-[3-chlor-4-(2-oxo-2*H-*pyridin-1-yl)-phenyl]-pentansäureamid,
2-(1-Amino-isochinolin-7-yloxy)-*N*-[4-(3-oxo-morpholin-4-yl)-phenyl]-4-methyl-pentansäureamid,
2-(1-Amino-isochinolin-7-yloxy)-*N*-[3-trifluormethyl-4-(3-oxo-morpholin-4-yl)-phenyl]-4-methyl-pentansäureamid,
2-(1-Amino-isochinolin-7-yloxy)-*N*-[3-trifluormethyl-4-(3-oxo-morpholin-4-yl)-phenyl]-pentansäureamid,
2-(1-Amino-isochinolin-7-ylamino)-*N*-[4-(2-oxo-2*H*-pyridin-1-yl)-phenyl]-4-methyl-pentansäureamid,
(S)-2-(1-Amino-isochinolin-7-ylamino)-*N*-[4-(3-oxo-morpholin-4-yl)-phenyl]-4-methyl-pentansäureamid,
2-(8-Oxo-5,6,7,8-tetrahydro-naphthalin-2-yloxy)-*N*-[3-methyl-4-(2-oxo-piperidin-1-yl)-phenyl]-2-phenyl-acetamid,
2-(8-Amino-5,6,7,8-tetrahydro-naphthalin-2-yloxy)-*N*-[3-methyl-4-(2-oxo-piperidin-1-yl)-phenyl]-2-phenyl-acetamid,
2-(8-Oxo-5,6,7,8-tetrahydro-naphthalin-2-yloxy)-*N*-[3-methyl-4-(2-oxo-piperidin-1-yl)-phenyl]-2-(2-fluorphenyl)-acetamid,
2-(Chinolin-6-ylamino)-*N*-[4-(2-oxo-2*H*-pyridin-1-yl)-phenyl]-2-phenyl-acetamid,
2-(Chinolin-6-ylamino)-*N*-[4-(2-oxo-piperidin-1-yl)-phenyl]-2-phenyl-acetamid,
2-(Chinolin-6-ylamino)-*N*-[3-methyl-4-(2-oxo-piperidin-1-yl)-phenyl]-2-phenyl-acetamid,
2-(Isochinolin-7-ylamino)-*N*-[4-(2-oxo-2*H*-pyridin-1-yl)-phenyl]-4-methyl-pentansäureamid,
2-(3-Amino-1*H*-indazol-5-yloxy)-*N*-[4-(3-oxo-morpholin-4-yl)-phenyl]-4-methyl-pentansäureamid,
2-(3-Amino-benzo[*d*]isoxazol-5-yloxy)-*N*-[4-(3-oxo-morpholin-4-yl)-phenyl]-4-methyl-pentansäureamid,
2-(3-Amino-benzo[*d*]isothiazol-5-ylamino)-*N*-[4-(3-oxo-morpholin-4-yl)-phenyl]-2-(2-fluorphenyl)-acetamid,
2-(3-Amino-benzo[*c*]isothiazol-5-yloxy)-*N*-[4-(3-oxo-morpholin-4-yl)-phenyl]-4-methyl-pentansäureamid,
2-(3-Amino-benzo[*d*]isothiazol-5-yloxy)-*N*-[4-(3-oxo-morpholin-4-yl)-phenyl]-4-methyl-pentansäureamid,
2-(3-Amino-1*H*-indazol-5-ylamino)-*N*-[4-(3-oxo-morpholin-4-yl)-phenyl]-4-methyl-pentansäureamid,
2-(3-Amino-benzo[*d*]isoxazol-5-ylamino)-*N*-[4-(3-oxo-morpholin-4-yl)-phenyl]-4-methyl-pentansäureamid,
sowie ihre pharmazeutisch verwendbaren Salze, Solvate und Stereoisomere, einschließlich deren Mischungen in allen Verhältnissen.

2. Arzneimittel, enthaltend mindestens eine Verbindung nach Anspruch 1 und/oder ihre pharmazeutisch verwendbaren Salze, Solvate und Stereoisomere, einschließlich deren Mischungen in allen Verhältnissen, sowie gegebenenfalls Träger- und/oder Hilfsstoffe.

3. Verwendung von Verbindungen nach Anspruch 1 und/oder ihre physiologisch unbedenklichen Salze und Solvate zur Herstellung eines Arzneimittels zur Behandlung von Thrombosen, myocardialem Infarkt, Arteriosklerose, Entzündungen, Apoplexie, Angina pectoris, Restenose nach Angioplastie, Claudicatio intermittens, Migräne, Tumoren, Tumorerkrankungen und/oder Tumormetastasen.

## Claims

1. Compounds selected from the group
2-(isoquinolin-7-yloxy)-*N*-[4-(2-oxopiperidin-1-yl)phenyl]-2-phenylacetamide,
2-(1-aminoisoquinolin-7-yloxy)-*N*-[4-(2-oxopiperidin-1-yl)-phenyl]-2-phenylacetamide,
2-(isoquinolin-7-yloxy)-*N*-[4-(2-oxopiperidin-1-yl)phenyl]-2-phenylacetamide,
2-(isoquinolin-7-yloxy)-*N*-[4-(2-oxopiperidin-1-yl)phenyl]-pentanamide,
2-(1-aminoisoquinolin-7-yloxy)-*N*-[4-(2-oxopiperidin-1-yl)-phenyl]acetamide,
2-(isoquinolin-7-yloxy)-*N*-[4-(2-oxopiperidin-1-yl)phenyl]-acetamide,
2-(isoquinolin-7-yloxy)-*N*-[4-(2-oxopyrrolidin-1-yl)-3-methylphenyl]pentanamide,
2-(isoquinolin-7-yloxy)-*N*-[4-(2-oxopiperidin-1-yl)-3-methylphenyl]pentanamide,
2-(isoquinolin-7-yloxy)-*N*-[4-(2-oxoazepan-1-yl)-3-methylphenyl]pentanamide,
2-(isoquinolin-7-yloxy)-*N*-[4-(2-oxopiperidin-1-yl)-3-methylphenyl]acetamide,
2-(2,1,3-benzothiadiazol-5-ylamino)-*N*-[4-(2-oxopiperidin-1-yl)-3-methylphenyl]-2-phenylacetamide,
2-(1,3-benzodioxol-5-ylamino)-*N*-[4-(2-oxopiperidin-1-yl)-3-methylphenyl]-2-phenylacetamide,
2-(indol-5-ylamino)-*N*-[4-(2-oxopiperidin-1-yl)-3-methylphenyl]-2-phenylacetamide,
2-(2-methylbenzimidazol-5-ylamino)-*N*-[4-(2-oxopiperidin-1-yl)-3-methylphenyl]-2-phenylacetamide,
2-(benzothiazol-6-ylamino)-*N*-[4-(2-oxopiperidin-1-yl)-3-methylphenyl]-2-phenylacetamide,
2-(isoquinolin-7-ylamino)-*N*-[4-(2-oxopiperidin-1-yl)-3-methylphenyl]-2-phenylacetamide,
2-(isoquinolin-6-ylamino)-*N*-[4-(2-oxopiperidin-1-yl)-3-methylphenyl]-2-phenylacetamide,
2-(1*H*-indazol-6-ylamino)-*N*-[4-(2-oxopiperidin-1-yl)-3-methylphenyl]-2-phenylacetamide,
2-(1*H*-indazol-5-ylamino)-*N*-[4-(2-oxopiperidin-1-yl)-3-methylphenyl]-2-phenylacetamide,
2-(2,3-dihydrobenzo-1,4-dioxin-6-ylamino)-*N*-[4-(2-oxopiperidin-1-yl)-3-methylphenyl]-2-phenylacetamide,
2-(quinoxalin-6-ylamino)-*N*-[4-(2-oxopiperidin-1-yl)-3-methylphenyl]-2-phenylacetamide,
2-(isoquinolin-7-yloxy)-*N*-[3-methyl-4-(2-oxopiperidin-1-yl)-phenyl]-2-(2-fluorophenyl)acetamide,
2-(isoquinolin-7-yloxy)-*N*-[4-(2-oxo-1*H*-pyrazin-1-yl)phenyl]-pentanamide,
(S)-2-(1-aminoisoquinolin-7-yloxy)-*N*-[4-(3-oxomorpholin-4-yl)phenyl]-4-methylpentanamide,
(S)-2-(1-aminoisoquinolin-7-yloxy)-*N*-[3-methyl-4-(3-oxo-morpholin-4-yl)phenyl]-4-methylpentanamide,
(S)-2-(1-aminoisoquinolin-7-yloxy)-*N*-[4-(2-oxo-2*H*-pyridin-1-yl)phenyl]-4-methylpentanamide,
2-(1-aminoisoquinolin-7-yloxy)-*N*-[4-(3-oxomorpholin-4-yl)-phenyl]pentanamide,
2-(1-aminoisoquinolin-7-yloxy)-*N*-[4-(3-oxomorpholin-4-yl)-phenyl]-2-(2-chlorophenyl)acetamide,
2-(1-aminoisoquinolin-7-yloxy)-*N*-[4-(3-oxo-2*H*-pyridazin-2-yl)-phenyl]-4-methylpentanamide,
2-(1-aminoisoquinolin-7-yloxy)-*N*-[4-(2-oxo-1*H*-pyrazin-1-yl)-phenyl]-4-methylpentanamide,
2-(1-aminoisoquinolin-7-yloxy)-*N*-[4-(2-oxopiperazin-1-yl)-phenyl]-4-methylpentanamide,
2-(1-aminoisoquinolin-7-yloxy)-*N*-[4-(2-oxo-1,3-oxazinan-3-yl)phenyl]-4-methylpentanamide,
2-(1-aminoisoquinolin-7-yloxy)-*N*-[4-(2-oxo-3*H*-thiazol-3-yl)-phenyl]-4-methylpentanamide,
2-(1-aminoisoquinolin-7-yloxy)-*N*-[4-(2-oxooxazolidin-3-yl)-phenyl]-4-methylpentanamide,
2-(1-aminoisoquinolin-7-yloxy)-*N*-[3-methyl-4-(2-oxo-oxazolidin-3-yl)phenyl]-4-methylpentanamide,
2-(1-aminoisoquinolin-7-yloxy)-*N*-[4-(2-oxotetrahydro-pyrimidin-1-yl)phenyl]-4-methylpentanamide,
2-(1-aminoisoquinolin-7-yloxy)-*N*-[4-(2-oxoimidazolidin-1-yl)-phenyl]-4-methylpentanamide,
2-(1-aminoisoquinolin-7-yloxy)-*N*-[3-methyl-4-(2-oxoazepan-1-yl)phenyl]pentanamide,
2-(1-aminoisoquinolin-7-yloxy)-*N*-[3-methyl-4-(2-oxopiperidin-1-yl)phenyl]pentanamide,
2-(1-aminoisoquinolin-7-yloxy)-*N*-[3-methyl-4-(2-oxopiperidin-1-yl)phenyl]acetamide,
2-(1-aminoisoquinolin-7-yloxy)-*N*-[3-trifluoromethyl-4-(2-aza-bicyclo[2.2.2]octan-3-on-2-yl)phenyl]pentanamide,
2-(1-aminoisoquinolin-7-yloxy)-*N*-[4-(2-oxopiperidin-1-yl)-phenyl]propionamide,
2-(1-aminoisoquinolin-7-yloxy)-*N*-[4-(2-oxopiperidin-1-yl)-phenyl]butyramide,
2-(1-aminoisoquinolin-7-yloxy)-*N*-[3-methyl-4-(2-oxopyrrolidin-1-yl)phenyl]butyramide,
2-(1-aminoisoquinolin-7-yloxy)-*N*-[4-(2-oxopiperidin-1-yl)-phenyl]pentanamide,
2-(1-aminoisoquinolin-7-yloxy)-*N*-[4-(2-oxopyrrolidin-1-yl)-phenyl]hexanamide,
2-(1-aminoisoquinolin-7-yloxy)-*N*-[3-methyl-4-(2-oxopiperidin-1-yl)phenyl]hexanamide,
2-(1-aminoisoquinolin-7-yloxy)-*N*-[4-(2-oxo-2*H*-pyridin-1-yl)-phenyl]butyramide,
2-(1-aminoisoquinolin-7-yloxy)-*N*-[4-(2-oxo-2*H*-pyridin-1-yl)-phenyl]pentanamide,
2-(1-aminoisoquinolin-7-yloxy)-*N*-[3-chloro-4-(2-oxopyrrolidin-1-yl)phenyl]pentanamide,
2-(1-aminoisoquinolin-7-yloxy)-*N*-[3-methyl-4-(2-oxopiperidin-1-yl)phenyl]butyramide,
2-(1-aminoisoquinolin-7-yloxy)-*N*-[4-(2-oxopiperidin-1-yl)-phenyl]hexanamide,
2-(1-aminoisoquinolin-7-yloxy)-*N*-[4-(2-oxopiperidin-1-yl)-phenyl]-4-methylpentanamide,
2-(1-aminoisoquinolin-7-yloxy)-*N*-[3-methyl-4-(2-oxopiperidin-1-yl)phenyl]-4-methylpentanamide, hydrochloride, ESI 461;
2-(1-aminoisoquinolin-7-yloxy)-*N*-[4-(2-oxo-2*H*-pyridin-1-yl)-phenyl]-4-methylpentanamide,
2-(1-aminoisoquinolin-7-yloxy)-*N*-[4-(2-oxopiperidin-1-yl)-phenyl]-2-(2-fluorophenyl)acetamide,
2-(1-aminoisoquinolin-7-yloxy)-*N*-[3-methyl-4-(2-oxopiperidin-1-yl)phenyl]-2-(2-fluorophenyl)acetamide,
(R)-2-(1-aminoisoquinolin-7-yloxy)-*N*-[4-(2-oxo-2*H*-pyridin-1-yl)phenyl]-4-methylpentanamide,
2-(1-aminoisoquinolin-7-yloxy)-*N*-[4-(2-oxo-2*H*-pyridin-1-yl)-phenyl]-4,4-dimethylpentanamide,
2-(1-aminoisoquinolin-7-yloxy)-*N*-[4-(2-oxo-2*H*-pyridin-1-yl)-phenyl]-2-(2-fluorophenyl)acetamide,
2-(1-aminoisoquinolin-7-yloxy)-*N*-[4-(3-oxomorpholin-4-yl)-phenyl]butyramide,
2-(1-aminoisoquinolin-7-yloxy)-*N*-[3-methyl-4-(3-oxo-morpholin-4-yl)phenyl]butyramide,
2-(1-aminoisoquinolin-7-yloxy)-*N*-[3-methyl-4-(3-oxo-morpholin-4-yl)phenyl]pentanamide,
2-(1-aminoisoquinolin-7-yloxy)-*N*-[3-chloro-4-(2-oxo-2*H-*pyridin-1-yl)phenyl]-4-methylpentanamide,
2-(1-aminoisoquinolin-7-yloxy)-*N*-[4-(3-oxomorpholin-4-yl)-phenyl]-2-(2-fluorophenyl)acetamide,
2-(1-aminoisoquinolin-7-yloxy)-*N*-[3-methyl-4-(3-oxo-morpholin-4-yl)phenyl]-4-methylpentanamide,
2-(1-aminoisoquinolin-7-yloxy)-*N*-[3-chloro-4-(2-oxopyrrolidin-1-yl)phenyl]-2-(2-fluorophenyl)acetamide,
2-(1-aminoisoquinolin-7-yloxy)-*N*-[3-chloro-4-(2-oxo-2H-pyridin-1-yl)phenyl]butyramide,
2-(1-aminoisoquinolin-7-yloxy)-*N*-[2-fluoro-4-(2-oxo-2*H-*pyridin-1-yl)phenyl]pentanamide,
2-(1-aminoisoquinolin-7-yloxy)-*N*-[2-methyl-4-(2-oxo-2*H-*pyridin-1-yl)phenyl]-4-methylpentanamide,
2-(1-aminoisoquinolin-7-yloxy)-*N*-[3-chloro-4-(2-oxo-2*H-*pyridin-1-yl)phenyl]pentanamide,
2-(1-aminoisoquinolin-7-yloxy)-*N*-[4-(3-oxomorpholin-4-yl)-phenyl]-4-methylpentanamide,
2-(1-aminoisoquinolin-7-yloxy)-*N*-[3-trifluoromethyl-4-(3-oxo-morpholin-4-yl)phenyl]-4-methylpentanamide,
2-(1-aminoisoquinolin-7-yloxy)-*N*-[3-trifluoromethyl-4-(3-oxo-morpholin-4-yl)phenyl]pentanamide,
2-(1-aminoisoquinolin-7-ylamino)-*N*-[4-(2-oxo-2*H*-pyridin-1-yl)phenyl]-4-methylpentanamide,
(S)-2-(1-aminoisoquinolin-7-ylamino)-*N*-[4-(3-oxomorpholin-4-yl)phenyl]-4-methylpentanamide,
2-(8-oxo-5,6,7,8-tetrahydronaphthalen-2-yloxy)-*N*-[3-methyl-4-(2-oxopiperidin-1-yl)phenyl]-2-phenylacetamide,
2-(8-amino-5,6,7,8-tetrahydronaphthalen-2-yloxy)-*N*-[3-methyl-4-(2-oxopiperidin-1-yl)phenyl]-2-phenylacetamide,
2-(8-oxo-5,6,7,8-tetrahydronaphthalen-2-yloxy)-*N*-[3-methyl-4-(2-oxopiperidin-1-yl)phenyl]-2-(2-fluorophenyl)acetamide,
2-(quinolin-6-ylamino)-*N*-[4-(2-oxo-2*H*-pyridin-1-yl)phenyl]-2-phenylacetamide,
2-(quinolin-6-ylamino)-*N*-[4-(2-oxopiperidin-1-yl)phenyl]-2-phenylacetamide,
2-(quinolin-6-ylamino)-*N*-[3-methyl-4-(2-oxopiperidin-1-yl)-phenyl]-2-phenylacetamide,
2-(isoquinolin-7-ylamino)-*N*-[4-(2-oxo-2*H*-pyridin-1-yl)phenyl]-4-methylpentanamide,
2-(3-amino-1*H*-indazol-5-yloxy)-*N*-[4-(3-oxomorpholin-4-yl)-phenyl]-4-methylpentanamide,
2-(3-aminobenzo[*d*]isoxazol-5-yloxy)-*N*-[4-(3-oxomorpholin-4-yl)phenyl]-4-methylpentanamide,
2-(3-aminobenzo[*d*]isothiazol-5-ylamino)-*N*-[4-(3-oxo-morpholin-4-yl)phenyl]-2-(2-fluorophenyl)acetamide,
2-(3-aminobenzo[*c*]isothiazol-5-yloxy)-*N*-[4-(3-oxomorpholin-4-yl)phenyl]-4-methylpentanamide,
2-(3-aminobenzo[*d*]isothiazol-5-yloxy)-*N*-[4-(3-oxomorpholin-4-yl)phenyl]-4-methylpentanamide,
2-(3-amino-1*H*-indazol-5-ylamino)-*N*-[4-(3-oxomorpholin-4-yl)phenyl]-4-methylpentanamide,
2-(3-aminobenzo[*d*]isoxazol-5-ylamino)-*N*-[4-(3-oxo-morpholin-4-yl)phenyl]-4-methylpentanamide,
and pharmaceutically usable salts, solvates and stereoisomers thereof, including mixtures thereof in all ratios.

2. Medicaments comprising at least one compound according to Claim 1 and/or pharmaceutically usable salts, solvates and stereoisomers thereof, including mixtures thereof in all ratios, and optionally excipients and/or adjuvants.

3. Use of compounds according to Claim 1 and/or physiologically acceptable salts and solvates thereof for the preparation of a medicament for the treatment of thromboses, myocardial infarction, arteriosclerosis, inflammation, apoplexy, angina pectoris, restenosis after angioplasty, claudicatio intermittens, migraine, tumours, tumour diseases and/or tumour metastases.

## Revendications

1. Composés choisis parmi le groupe
le 2-(isoquinolin-7-yloxy)-*N*-[4-(2-oxopipéridin-1-yl)phényl]-2-phénylacétamide,
le 2-(1-aminoisoquinolin-7-yloxy)-*N*-[4-(2-oxopipéridin-1-yl)-phényl]-2-phénylacétamide,
le 2-(isoquinolin-7-yloxy)-*N*-[4-(2-oxopipéridin-1-yl)phényl]-2-phénylacétamide,
le 2-(isoquinolin-7-yloxy)-*N*-[4-(2-oxopipéridin-1-yl)phényl]-pentanamide,
le 2-(1-aminoisoquinolin-7-yloxy)-*N*-[4-(2-oxopipéridin-1-yl)-phényl]acétamide,
le 2-(isoquinolin-7-yloxy)-*N*-[4-(2-oxopipéridin-1-yl)phényl]-acétamide,
le 2-(isoquinolin-7-yloxy)-*N*-[4-(2-oxopyrrolidin-1-yl)-3-méthyl-phényl]pentanamide,
le 2-(isoquinolin-7-yloxy)-*N*-[4-(2-oxopipéridin-1-yl)-3-méthyl-phényl]pentanamide,
le 2-(isoquinolin-7-yloxy)-*N*-[4-(2-oxoazepan-1-yl)-3-méthyl-phényl]pentanamide,
le 2-(isoquinolin-7-yloxy)-*N*-[4-(2-oxopipéridin-1-yl)-3-méthyl-phényl]acétamide,
le 2-(2,1,3-benzothiadiazol-5-ylamino)-*N*-[4-(2-oxopipéridin-1-yl)-3-méthylphényl]-2-phénylacétamide,
le 2-(1,3-benzodioxol-5-ylamino)-*N*-[4-(2-oxopipéridin-1-yl)-3-méthylphényl]-2-phénylacétamide,
le 2-(indol-5-ylamino)-*N*-[4-(2-oxopipéridin-1-yl)-3-méthyl-phényl]-2-phénylacétamide,
le 2-(2-méthylbenzimidazol-5-ylamino)-*N*-[4-(2-oxopipéridin-1-yl)-3-méthylphényl]-2-phénylacétamide,
le 2-(benzothiazol-6-ylamino)-*N*-[4-(2-oxopipéridin-1-yl)-3-méthylphényl]-2-phénylacétamide,
le 2-(isoquinolin-7-ylamino)-*N*-[4-(2-oxopipéridin-1-yl)-3-méthylphényl]-2-phénylacétamide,
le 2-(isoquinolin-6-ylamino)-*N*-[4-(2-oxopipéridin-1-yl)-3-méthylphényl]-2-phénylacétamide,
le 2-(1*H*-indazol-6-ylamino)-*N*-[4-(2-oxopipéridin-1-yl)-3-méthylphényl]-2-phénylacétamide,
le 2-(1*H*-indazol-5-ylamino)-*N*-[4-(2-oxopipéridin-1-yl)-3-méthylphényl]-2-phénylacétamide,
le 2-(2,3-dihydrobenzo-1,4-dioxin-6-ylamino)-*N*-[4-(2-oxo-pipéridin-1-yl)-3-méthylphényl]-2-phénylacétamide,
le 2-(quinoxalin-6-ylamino)-*N*-[4-(2-oxopipéridin-1-yl)-3-méthylphényl]-2-phénylacétamide,
le 2-(isoquinolin-7-yloxy)-*N*-[3-méthyl-4-(2-oxopipéridin-1-yl)-phényl]-2-(2-fluorophényl)acétamide,
le 2-(isoquinolin-7-yloxy)-*N*-[4-(2-oxo-1*H*-pyrazin-1-yl)phényl]-pentanamide,
le (S)-2-(1-aminoisoquinolin-7-yloxy)-*N*-[4-(3-oxomorpholin-4-yl)phényl]-4-méthylpentanamide,
le (S)-2-(1-aminoisoquinolin-7-yloxy)-*N*-[3-méthyl-4-(3-oxo-morpholin-4-yl)phényl]-4-méthylpentanamide,
le (S)-2-(1-aminoisoquinolin-7-yloxy)-*N*-[4-(2-oxo-2*H*-pyridin-1-yl)phényl]-4-méthylpentanamide,
le 2-(1-aminoisoquinolin-7-yloxy)-*N*-[4-(3-oxomorpholin-4-yl)-phényl]pentanamide,
le 2-(1-aminoisoquinolin-7-yloxy)-*N*-[4-(3-oxomorpholin-4-yl)-phényl]-2-(2-chlorophényl)acétamide,
le 2-(1-aminoisoquinolin-7-yloxy)-*N*-[4-(3-oxo-2*H*-pyridazin-2-yl)phényl]-4-méthylpentanamide,
le 2-(1-aminoisoquinolin-7-yloxy)-*N*-[4-(2-oxo-1*H*-pyrazin-1-yl)phényl]-4-méthylpentanamide,
le 2-(1-aminoisoquinolin-7-yloxy)-*N*-[4-(2-oxopiperazin-1-yl)-phényl]-4-méthylpentanamide,
le 2-(1-aminoisoquinolin-7-yloxy)-*N*-[4-(2-oxo-1,3-oxazinan-3-yl)phényl]-4-méthylpentanamide,
le 2-(1-aminoisoquinolin-7-yloxy)-*N*-[4-(2-oxo-3*H*-thiazol-3-yl)-phényl]-4-méthylpentanamide,
le 2-(1-aminoisoquinolin-7-yloxy)-*N*-[4-(2-oxooxazolidin-3-yl)-phényl]-4-méthylpentanamide,
le 2-(1-aminoisoquinolin-7-yloxy)-*N*-[3-méthyl-4-(2-oxo-oxazolidin-3-yl)phényl]-4-méthylpentanamide,
le 2-(1-aminoisoquinolin-7-yloxy)-*N*-[4-(2-oxotetrahydro-pyrimidin-1-yl)phényl]-4-méthylpentanamide,
le 2-(1-aminoisoquinolin-7-yloxy)-*N*-[4-(2-oxoimidazolidin-1-yl)phényl]-4-méthylpentanamide,
le 2-(1-aminoisoquinolin-7-yloxy)-*N*-[3-méthyl-4-(2-oxoazepan-1-yl)phényl]pentanamide,
le 2-(1-aminoisoquinolin-7-yloxy)-*N*-[3-méthyl-4-(2-oxo-pipéridin-1-yl)phényl]pentanamide,
le 2-(1-aminoisoquinolin-7-yloxy)-*N*-[3-méthyl-4-(2-oxo-pipéridin-1-yl)phényl]acétamide,
le 2-(1-aminoisoquinolin-7-yloxy)-*N*-[3-trifluorométhyl-4-(2-azabicyclo[2.2.2]octan-3-on-2-yl)phényl]pentanamide,
le 2-(1-aminoisoquinolin-7-yloxy)-*N*-[4-(2-oxopipéridin-1-yl)-phényl]propionamide,
le 2-(1-aminoisoquinolin-7-yloxy)-*N*-[4-(2-oxopipéridin-1-yl)-phényl]butyramide,
le 2-(1-aminoisoquinolin-7-yloxy)-*N*-[3-méthyl-4-(2-oxo-pyrrolidin-1-yl)phényl]butyramide,
le 2-(1-aminoisoquinolin-7-yloxy)-*N*-[4-(2-oxopipéridin-1-yl)-phényl]pentanamide,
le 2-(1-aminoisoquinolin-7-yloxy)-*N*-[4-(2-oxopyrrolidin-1-yl)-phényl]hexanamide,
le 2-(1-aminoisoquinolin-7-yloxy)-*N*-[3-méthyl-4-(2-oxo-pipéridin-1-yl)phényl]hexanamide,
le 2-(1-aminoisoquinolin-7-yloxy)-*N*-[4-(2-oxo-2*H*-pyridin-1-yl)-phényl]butyramide,
le 2-(1-aminoisoquinolin-7-yloxy)-*N*-[4-(2-oxo-2*H*-pyridin-1-yl)-phényl]pentanamide,
le 2-(1-aminoisoquinolin-7-yloxy)-*N*-[3-chloro-4-(2-oxo-pyrrolidin-1-yl)phényl]pentanamide,
le 2-(1-aminoisoquinolin-7-yloxy)-*N*-[3-méthyl-4-(2-oxo-pipéridin-1-yl)phényl]butyramide,
le 2-(1-aminoisoquinolin-7-yloxy)-*N*-[4-(2-oxopipéridin-1-yl)-phényl]hexanamide,
le 2-(1-aminoisoquinolin-7-yloxy)-*N*-[4-(2-oxopipéridin-1-yl)-phényl]-4-méthylpentanamide,
l'hydrochlorure de 2-(1-aminoisoquinolin-7-yloxy)-*N*-[3-méthyl-4-(2-oxopipéridin-1-yl)phényl]-4-méthylpentanamide, ESI 461,
le 2-(1-aminoisoquinolin-7-yloxy)-*N*-[4-(2-oxo-2*H*-pyridin-1-yl)-phényl]-4-méthylpentanamide,
le 2-(1-aminoisoquinolin-7-yloxy)-*N*-[4-(2-oxopipéridin-1-yl)-phényl]-2-(2-fluorophényl)acétamide,
le 2-(1-aminoisoquinolin-7-yloxy)-*N*-[3-méthyl-4-(2-oxo-pipéridin-1-yl)phényl]-2-(2-fluorophényl)acétamide,
le (R)-2-(1-aminoisoquinolin-7-yloxy)-*N*-[4-(2-oxo-2*H*-pyridin-1-yl)phényl]-4-méthylpentanamide,
le 2-(1-aminoisoquinolin-7-yloxy)-*N*-[4-(2-oxo-2*H*-pyridin-1-yl)-phényl]-4,4-diméthylpentanamide,
le 2-(1-aminoisoquinolin-7-yloxy)-*N*-[4-(2-oxo-2*H*-pyridin-1-yl)-phényl]-2-(2-fluorophényl)acétamide,
le 2-(1-aminoisoquinolin-7-yloxy)-*N*-[4-(3-oxomorpholin-4-yl)-phényl]butyramide,
le 2-(1-aminoisoquinolin-7-yloxy)-*N*-[3-méthyl-4-(3-oxo-morpholin-4-yl)phényl]butyramide,
le 2-(1-aminoisoquinolin-7-yloxy)-*N*-[3-méthyl-4-(3-oxo-morpholin-4-yl)phényl]pentanamide,
le 2-(1-aminoisoquinolin-7-yloxy)-*N*-[3-chloro-4-(2-oxo-2*H-*pyridin-1-yl)phényl]-4-méthylpentanamide,
le 2-(1-aminoisoquinolin-7-yloxy)-*N*-[4-(3-oxomorpholin-4-yl)-phényl]-2-(2-fluorophényl)acétamide,
le 2-(1-aminoisoquinolin-7-yloxy)-*N*-[3-méthyl-4-(3-oxo-morpholin-4-yl)phényl]-4-méthylpentanamide,
le 2-(1-aminoisoquinolin-7-yloxy)-*N*-[3-chloro-4-(2-oxo-pyrrolidin-1-yl)phényl]-2-(2-fluorophényl)acétamide,
le 2-(1-aminoisoquinolin-7-yloxy)-*N*-[3-chloro-4-(2-oxo-2*H-*pyridin-1-yl)phényl]butyramide,
le 2-(1-aminoisoquinolin-7-yloxy)-*N*-[2-fluoro-4-(2-oxo-2*H-*pyridin-1-yl)phényl]pentanamide,
le 2-(1-aminoisoquinolin-7-yloxy)-*N*-[2-méthyl-4-(2-oxo-2*H-*pyridin-1-yl)phényl]-4-méthylpentanamide,
le 2-(1-aminoisoquinolin-7-yloxy)-*N*-[3-chloro-4-(2-oxo-2*H-*pyridin-1-yl)phényl]pentanamide,
le 2-(1-aminoisoquinolin-7-yloxy)-*N*-[4-(3-oxomorpholin-4-yl)-phényl]-4-méthylpentanamide,
le 2-(1-aminoisoquinolin-7-yloxy)-*N*-[3-trifluorométhyl-4-(3-oxomorpholin-4-yl)phényl]-4-méthylpentanamide,
le 2-(1-aminoisoquinolin-7-yloxy)-*N*-[3-trifluorométhyl-4-(3-oxomorpholin-4-yl)phényl]pentanamide,
le 2-(1-aminoisoquinolin-7-ylamino)-*N*-[4-(2-oxo-2*H*-pyridin-1-yl)phényl]-4-méthylpentanamide,
le (S)-2-(1-aminoisoquinolin-7-ylamino)-*N*-[4-(3-oxo-morpholin-4-yl)phényl]-4-méthylpentanamide,
le 2-(8-oxo-5,6,7,8-tetrahydronaphthalen-2-yloxy)-*N*-[3-méthyl-4-(2-oxopipéridin-1-yl)phényl]-2-phénylacétamide,
le 2-(8-amino-5,6,7,8-tetrahydronaphthalen-2-yloxy)-*N*-[3-méthyl-4-(2-oxopipéridin-1-yl)phényl]-2-phénylacétamide,
le 2-(8-oxo-5,6,7,8-tetrahydronaphthalen-2-yloxy)-*N*-[3-méthyl-4-(2-oxopipéridin-1-yl)phényl]-2-(2-fluorophényl)acétamide,
le 2-(quinolin-6-ylamino)-*N*-[4-(2-oxo-2*H*-pyridin-1-yl)phényl]-2-phénylacétamide,
le 2-(quinolin-6-ylamino)-*N*-[4-(2-oxopipéridin-1-yl)phényl]-2-phénylacétamide,
le 2-(quinolin-6-ylamino)-*N*-[3-méthyl-4-(2-oxopipéridin-1-yl)-phényl]-2-phénylacétamide,
le 2-(isoquinolin-7-ylamino)-*N*-[4-(2-oxo-2*H*-pyridin-1-yl)-phényl]-4-méthylpentanamide,
le 2-(3-amino-1*H*-indazol-5-yloxy)-*N*-[4-(3-oxomorpholin-4-yl)-phényl]-4-méthylpentanamide,
le 2-(3-aminobenzo[*d*]isoxazol-5-yloxy)-*N*-[4-(3-oxomorpholin-4-yl)phényl]-4-méthylpentanamide,
le 2-(3-aminobenzo[*d*]isothiazol-5-ylamino)-*N*-[4-(3-oxo-morpholin-4-yl)phényl]-2-(2-fluorophényl)acétamide,
le 2-(3-aminobenzo[*c*]isothiazol-5-yloxy)-*N*-[4-(3-oxo-morpholin-4-yl)phényl]-4-méthylpentanamide,
le 2-(3-aminobenzo[*d*]isothiazol-5-yloxy)-*N*-[4-(3-oxo-morpholin-4-yl)phényl]-4-méthylpentanamide,
le 2-(3-amino-1*H*-indazol-5-ylamino)-*N*-[4-(3-oxomorpholin-4-yl)phényl]-4-méthylpentanamide,
le 2-(3-aminobenzo[*d*]isoxazol-5-ylamino)-*N*-[4-(3-oxo-morpholin-4-yl)phényl]-4-méthylpentanamide,
et les sels, solvats et stéréoisomères pharmaceutiquement utilisables de ceux-ci, y compris des mélanges de ceux-ci en toutes proportions.

2. Médicaments comprenant au moins un composé selon la revendication 1 et/ou des sels, solvats et stéréoisomères pharmaceutiquement utilisables de celui-ci, y compris des mélanges de ceux-ci en toutes proportions, et éventuellement des excipients et/ou des adjuvants

3. Utilisation de composés selon la revendication 1 et/ou de sels et solvats physiologiquement acceptables de ceux-ci pour la préparation d'un médicament destiné au traitement des thromboses, de l'infarctus du myocarde, de l'artériosclérose, des inflammations, de l'apoplexie, de l'angine de poitrine, de la resténose post-angioplastie, de la boiterie intermittente, de la migraine, des tumeurs, des maladies tumorales et/ou des métastases tumorales.
